# EUROPEAN PATENT SPECIFICATION

(11) **EP 2 176 294 B1**
(45) Date of publication and mention of the grant of the patent: **20.05.2015**
(21) Application number: 08771835.9
(22) Date of filing: 24.06.2008
(51) Int. Cl.: C07K 16/28, C07K 14/705, A61K 39/395, A61P 19/08

(54) **USES OF MDL-1 ANTAGONISTS**
VERWENDUNGEN VON MDL-1 ANTAGONISTEN
UTILISATION D'ANTAGONISTES DE MDL-1

(30) Priority: 29.06.2007 US 947314 P
(43) Date of publication of application: 21.04.2010
(73) Proprietor: Merck Sharp & Dohme Corp., Rahway, NJ 07065-0907 (US)
(72) Inventor: BIGLER, Michael, E., Redwood City, California 94062 (US); CUA, Daniel, J., Boulder Creek, California 95006 (US); JOYCE-SHAIKH, Barbara, San Jose, California 95125 (US); PHILLIPS, Joseph, H., Palo Alto, California 94303 (US)
(74) Representative: Horgan, James Michael Frederic
(86) International application number: PCT/US2008/068042
(87) International publication number: WO 2009/006112

(56) References cited:
- WO-A-2007/088051
- KOGA TAKAKO ET AL: "Costimulatory signals mediated by the ITAM motif cooperate with RANKL for bone homeostasis" NATURE (LONDON), vol. 428, no. 6984, 15 April 2004 (2004-04-15), pages 758-763, XP009105265 ISSN: 0028-0836
- KAIFU TOMONORI ET AL: "Osteopetrosis and thalamic hypomyelinosis with synaptic degeneration in DAP12-deficient mice" JOURNAL OF CLINICAL INVESTIGATION, AMERICAN SOCIETY FOR CLINICAL INVESTIGATION, US, vol. 111, no. 3, 1 February 2003 (2003-02-01), pages 323-332, XP002412551 ISSN: 0021-9738
- HUMPHREY MARY BETH ET AL: "The signaling adapter protein DAP12 regulates multinucleation during osteoclast development" JOURNAL OF BONE AND MINERAL RESEARCH, NEW YORK, NY, US, vol. 19, no. 2, 1 February 2004 (2004-02-01), pages 224-234, XP002413095 ISSN: 0884-0431
- HUMPHREY MARY BETH ET AL: "TREM2, a DAP12-associated receptor, regulates osteoclast differentiation and function" JOURNAL OF BONE AND MINERAL RESEARCH, vol. 21, no. 2, February 2006 (2006-02), pages 237-245, XP009105217 ISSN: 0884-0431
- COLONNA MARCO ET AL: "The enigmatic function of TREM-2 in osteoclastogenesis" ADVANCES IN EXPERIMENTAL MEDICINE AND BIOLOGY, SPRING ST., NY, vol. 602, 1 January 2007 (2007-01-01), pages 97-105, XP009105054 ISSN: 0065-2598
- TURNBULL ISAIAH R ET AL: "Activating and inhibitory functions of DAP12" NATURE REVIEWS. IMMUNOLOGY, XX, XX, vol. 7, no. 2, 1 February 2007 (2007-02-01), pages 155-161, XP009105068 ISSN: 1474-1733
- BAKKER A B ET AL: "Myeloid DAP12-associating lectin (MDL)-1 is a cell surface receptor involved in the activation of myeloid cells" PROCEEDINGS OF THE NATIONAL ACADEMY OF SCIENCES OF USA, NATIONAL ACADEMY OF SCIENCE, WASHINGTON, DC, vol. 96, no. 17, 17 August 1999 (1999-08-17), pages 9792-9796, XP009105045 ISSN: 0027-8424

## Description

### FIELD OF THE INVENTION

The invention relates to methods for treating skeletal and immune disorders by modulation of MDL-1 activity.

### BACKGROUND OF THE INVENTION

Bone tissue is primarily composed of three cell types (osteoblasts, osteocytes, and osteoclasts) and a mineralized intercellular bone matrix comprising polymers (primarily collagen fibers) and other organic substances (ground substance, composed primarily of proteoglycans such as chondroitin sulfate and hyaluronic acid) synthesized by bone cells (primarily osteoblasts). Bone cells produce the organic molecules of bone matrix and also modulate its mineralization. Osteoblasts are located at bone tissue surfaces and synthesize the organic components of the bone matrix. Osteocytes are mature osteoblasts and are involved in maintaining the bone matrix. Osteoclasts are involved in bone erosion and resorption.

The balance between osteoblast and osteoclast differentiation is critical for bone homeostasis. Dysregulation of this balance can lead to excessive osteoclast activation and bone resorptive diseases such as osteoporosis and osteoarthritis. Receptor activator of NF-kB ligand (RANKL)-which activates TRAF6, c-Fos, and NFATc1 transcriptional factors-is an important regulatory cytokine that provide primary signals for osteoclast development and function. Additional signals derived from immunoreceptor tyrosine-based activation motif (ITAM)-containing molecules are also essential for in vivo osteoclastogenesis.

DAP 12 (DNAX Activating Protein) is a disulfide-bonded, homodimeric type I transmembrane glycoprotein containing an immunoreceptor tyrosine-based activation motif (ITAM) located in its intracellular domain (Lanier, et al. (1998) Nature 391:703-707; WO 99/06557; Campbell and Colonna (1999) Int. J. Biochem. Cell Biol. 31:631-636; Lanier and Bakker (2000) Immunol. Today 21:611-614). The importance of DAP12 relies on the ITAM domain (Gingras et al. (2001) Mol. Immun. 38:817-824). Because the intracellular domain of the receptors of the Ig superfamily *(*Bouchon et al. (2000) J. Immunol. 164: 4991-4995; Dietrich et al. (2000) J. Immunol. 164:9-12) and the C-type lectin superfamily (Bakker et al. (1999) PNAS U.S.A. 96:9792-9796) that non-covalently associate with DAP12 are too short to allow interaction with other molecules, the DAP12 cytoplasmic domain constitutes the signaling subunit of these receptor complexes. Upon engagement of the receptor ligand-binding subunit, the DAP12 cytoplasmic ITAM is phosphorylated by Src kinases. The ITAM of DAP12 then interacts with Syk cytoplasmic tyrosine kinases, which initiates a cascade of events that leads to activation *(Lanier et al., supra*; Campbell and Colonna, *supra*; Lanier and Bakker, *supra*).

DAP 12 is expressed in monocytes, macrophages, natural killer (NK) cells, granulocytes, dendritic cells and mast cells, where it provides signaling function for at least eight distinct receptors *(*Gingras et al. (2001) Mol. Immun. 38:817-824; Lanier and Bakker, (2000) Immunol. Today 21:611-614). The myeloid receptor of the C-type lectin superfamily associated with DAP 12 is Myeloid DAP12-associating Lectin-1 (MDL-1), a type II transmembrane protein (MDL-1 is also referred to as CLEC5a). MDL-1 was the first DAP 12 associating molecule to be identified and cloned (Bakker et al. (1999) PNAS USA 96(17):9792-9796). It is expressed exclusively in monocytes and macrophages *(*Bakker et al. (1999) PNAS U.S.A. 96:9792-9796) as well as on other myeloid cell types such as, neutrophils and dendritic cells. The presence of a negatively charged residue in the transmembrane domain of DAP12 precludes its cell surface expression in the absence of a partner receptor, such as MDL-1, which has a positively charged residue in its transmembrane domain. However, DAP 12 alone is not sufficient for its expression and function at the cell surface. Thus, the combination of a DAP12-associating molecule, such as MDL-1, and DAP 12 may account for transmitting a particular physiological signal via DAP12 (Nochi et al. (2003) Am. J. of Pathology 162:1191-1201).

Recent studies have shown that costimulatory signals mediated by the DAP12-ITAM signaling pathway are required for osteoclast development (see, e.g., Koga, et al. (2004) Nature 428:758-763); *Dap12-*/*-* mice have an osteoclast development defect (see, e.g., Humphrey, et al. (2004) JBone Miner Res 19:224-234); inactivation of DAP-12 can result in wrist and ankle bone cysts and dementia (see, e.g., Paloneva, et al. (2002) Nature Genetics 25:357-361; and Paloneva, et al. (2001) Neurology 56:1552-1558).

Engagement of MDL-1 by the virus causing Dengue fever has been shown to induce DAP-12 phosphorylation and stimulates the release of proinflammatory cytokines (see, Chen et al. (2008) Nature*,* online publication doi:10.1038/nature07013:1-7). Interestingly, Dengue fever has also been called "Break-bone fever" due to the angonizing limb pain associated with active viral infection (see ,e.g., Clarke (2002) Nature 416:672-674).

WO-A-2007088051 (Bayer Schering Pharma Aktiengesellschaft) published after the priority date discloses an MDL-1 fusion protein that modulates MDL-1 activity for treating inflammatory diseases such as arthritis, multiple sclerosis and inflammatory bowel disease.

Inflammation normally is a localized, protective response to trauma or microbial invasion that destroys, dilutes, or walls-off the injurious agent and the injured tissue. It is characterized in the acute form by the classic signs of pain, heat, redness, swelling, and loss of function. Microscopically, it involves a complex series of events, including dilation of arterioles, capillaries, and venules, with increased permeability and blood flow, exudation of fluids, including plasma proteins, and leukocyte migration into the area of inflammation.

It has become increasingly clear that merely stopping inflammation might not be adequate for treatment of the resulting bone metabolism dysregulation. Therefore therapeutic strategies need to incorporate both anti-bone-resorptive and anti-inflammatory activities. The present invention fills this need by providing agents and methods for modulating bone density disorders by targeting MDL-1 activity in order to reverse bone resorption and suppress inflammation simultaneously.

### BRIEF DESCRIPTION OF THE DRAWINGS

Figure 1 shows that activation of MDL-1 augments the LPS induced release of inflammatory mediators.
Figure 2 shows exacerbation of CIA with administration of the MDL-1 agonist antibody, DX163.
Figure 3 shows inhibition of CAIA by the MDL-1 antagonist MDL-1-Ig fusion protein.
Figure 4 shows lower clinical scores of CAIA in MDL-1 KO mice.
Figure 5 confirms that agonizing MDL-1 activity with the agonist MDL-1 antibody, DX163, can exacerbate development of autoimmune arthritis.
Figure 6 shows inhibition of CAIA with MDL-1-Ig fusion protein.
Figure 7 shows paws from B10RIII mice that were scanned with GE explore CT scanner.
Figure 8 shows mRNA expression levels of genes associated with bone destruction such as RANKL, matrix metaloprotease 9 (MMP9) and TRAP were up-regulated in paws after anti-MDL-1 agonist treatment and down-regulated in MDL-1-Ig fusion treatment.
Figure 9 shows that treatment with RANK-L in combination with anti-MDL-1 agonist antibody increases expression of the osteoclast "master transcription regulator" NFATc1.

### SUMMARY OF THE INVENTION

The present invention is based upon the discovery that antagonizing MDL-1 activity inhibited bone erosion and inflammation. Provided is an antibody or antibody fragment thereof that specifically binds MDL-1 (SEQ ID NO:2 or 4) or a soluble MDL-1 protein (SEQ ID NO:2 or 4), for use in the diagnosis or treatment of a disease characterized by bone loss. In certain embodiments, the antibody is humanized, fully human, or chimeric. The antibody fragment is a Fab, Fab2, or Fv antibody fragment. The antibody or antibody fragment thereof can be conjugated to another chemical moiety, including polyethylene glycol (PEG). The antibody or antibody fragment inhibits bone resorption, including bone resorption caused by inflammation. The antibody or antibody fragment further inhibits osteoclast formation or activation.

In certain embodiments, the soluble MDL-1 protein is conjugated to a chemical moiety, including PEG. In further embodiments, the soluble MDL-1 protein is fused to a heterologous protein, including an Fc portion of an antibody molecule. The soluble MDL-1 protein inhibits bone resorption, including bone resorption caused by inflammation. The soluble MDL-1 protein further inhibits osteoclast formation or activation.

### DEFINITIONS

As used herein, the term "white blood cell" refers to a blood cell that does not contain hemoglobin. A white blood cell is also called a leukocyte. White blood cells include lymphocytes, neutrophils, eosinophils, monocytes, macrophages and mast cells.

As used herein, the term "expression status" is used to broadly refer to the variety of factors involved in the expression, function and regulation of a gene and its products, such as the level of mRNA expression, the integrity of the expressed gene products (such as the nucleic and amino acid sequences), and transcriptional and translational modifications to these molecules.

As used herein, the term "antibody molecule" refers to whole antibodies *(e.g.,* IgG, preferably, IgG1 or IgG4) and fragments, preferably antigen-binding fragments, thereof. Antibody fragments include Fab antibody fragments, F(ab)2 antibody fragments, Fv antibody fragments, single chain Fv antibody fragments and dsFv antibody fragments.

As used herein, the term "subject" or "patient" or "host" refers to any organism, preferably an animal, more preferably a mammal (e.g., mouse, rat, rabbit, cow, dog, cat, cow, chimpanzee, gorilla) and most preferably a human.

As used herein, the term "control" includes; a patient without an immune disorder; a sample from a patient without an immune disorder; a non diseased sample from a patient with an immune disorder.

As used herein, the terms "administration" and "treatment" as it applies to an animal, human, experimental subject, cell, tissue, organ, or biological fluid, refers to contact of an exogenous pharmaceutical, therapeutic, diagnostic agent, compound, or composition to the animal, human, subject, cell, tissue, organ, or biological fluid. "Administration" and "treatment" also *means in vitro*, *in vivo* and *ex vivo* treatments.

As used herein, the term "therapeutically effective amount" of a therapeutic agent is defined as an amount of each active component of the pharmaceutical formulation that is sufficient to show a meaningful patient benefit, *i.e.,* to cause a decrease in, prevention, or amelioration of the symptoms of the condition being treated. When the pharmaceutical formulation comprises a diagnostic agent, "a therapeutically effective amount" is defined as an amount that is sufficient to produce a signal, image, or other diagnostic parameter. Effective amounts of the pharmaceutical formulation will vary according to factors such as the degree of susceptibility of the individual, the age, gender, and weight of the individual, and idiosyncratic responses of the individual, see, *e.g.,* U.S. Pat. No. 5,888,530.

As used herein, the term "exogenous" refers to substances that are produced outside an organism, cell, or human body, depending on the context. As used herein, the term "endogenous" refers to substances that are produced within a cell, organism, or human body, depending on the context.

As used herein, the term "recombinant" refers to two or more nucleic acids or proteins which are not naturally contiguous and which are fused to each other. The term may also refer to a nucleic acid or protein which has been altered (e.g., post-translationally modified or mutated) by human intervention. For example, a wild-type codon may be replaced with a redundant codon encoding the same amino acid residue or a conservative substitution, while at the same time introducing or removing a nucleic acid sequence recognition site. Similarly, nucleic acid segments encoding desired functions may be fused to generate a single genetic entity encoding a desired combination of functions not found together in nature. Although restriction enzyme recognition sites are often the targets of such artificial manipulations, other site-specific targets, *e.g.*, promoters, DNA replication sites, regulation sequences, control sequences, or other useful features may be incorporated by design. Sequences encoding epitope tags for detection or purification, as described below, may also be incorporated.

As used herein, the term "polynucleotide", "nucleic acid" or "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in single stranded form, double-stranded form or otherwise.

As used herein, the term "polynucleotide sequence", "nucleic acid sequence" or "nucleotide sequence" refers to a series of nucleotide bases (also called "nucleotides") in a nucleic acid, such as DNA or RNA, and means any chain of two or more nucleotides.

As used herein, the term "coding sequence" or a sequence "encoding" refers to an expression product, such as a RNA, polypeptide, protein, or enzyme, is a nucleotide sequence that, when expressed, results in production of the product.

As used herein, the term "gene" means a DNA sequence that codes for or corresponds to a particular sequence of ribonucleotides or amino acids which comprise all or part of one or more RNA molecules, proteins or enzymes, and may or may not include regulatory DNA sequences, such as promoter sequences, which determine, for example, the conditions under which the gene is expressed. Genes may be transcribed from DNA to RNA which may or may not be translated into an amino acid sequence.

As used herein, the term "amplification" of DNA refers to the use of polymerase chain reaction (PCR) to increase the concentration of a particular DNA sequence within a mixture of DNA sequences. For a description of PCR see Saiki, et al., Science (1988) 239: 487.

As used herein, the term "oligonucleotide" refers to a nucleic acid, generally of at least 10 *e.g.,* 10, 11, 12, 13 or 14, preferably at least 15 *e.g.,* 15, 16, 17, 18 or 19, and more preferably at least 20 nucleotides e.g., 20, 21, 22, 23, 24, 25, 26, 27, 28, 29 or 30, preferably no more than 100 nucleotides e.g., 40, 50, 60, 70, 80 or 90, that may be hybridizable to a genomic DNA molecule, a cDNA molecule, or an mRNA molecule encoding a gene, mRNA, cDNA, or other nucleic acid of interest. Oligonucleotides may be labeled, e.g., by incorporation of ³²P-nucleotides, ³H-nucleotides, ¹⁴C-nucleotides, ³⁵S-nucleotides or nucleotides to which a label, such as biotin, has been covalently conjugated. In one embodiment, a labeled oligonucleotide may be used as a probe to detect the presence of a nucleic acid. In another embodiment, oligonucleotides (one or both of which may be labeled) may be used as PCR primers, either for cloning full length or a fragment of the gene, or to detect the presence of nucleic acids. Generally, oligonucleotides are prepared synthetically, preferably on a nucleic acid synthesizer.

As used herein, the term "promoter" or "promoter sequence" refers to a DNA regulatory region capable of binding an RNA polymerase in a cell (e.g., directly or through other promoter-bound proteins or substances) and initiating transcription of a coding sequence. A promoter sequence is, in general, bounded at its 3' terminus by the transcription initiation site and extends upstream (5' direction) to include the minimum number of bases or elements necessary to initiate transcription at any level. Within the promoter sequence may be found a transcription initiation site (conveniently defined, for example, by mapping with nuclease S1), as well as protein binding domains (consensus sequences) responsible for the binding of RNA polymerase. The promoter may be operably associated with other expression control sequences, including enhancer and repressor sequences or with a nucleic acid of the invention.

As used herein, the terms "express" and "expression" mean allowing or causing the information in a gene, RNA or DNA sequence to become manifest; for example, producing a protein by activating the cellular functions involved in transcription and translation of a corresponding gene. A DNA sequence is expressed in or by a cell to form an "expression product" such as an RNA *(e.g.,* mRNA) or a protein *(e.g.,* antibody or a fragment thereof). The expression product itself may also be said to be "expressed" by the cell.

As used herein, the terms "vector", "cloning vector" and "expression vector" mean the vehicle *(e.g.,* a plasmid) by which a DNA or RNA sequence may be introduced into a host cell, so as to transform the host and, optionally, promote expression and/or replication of the introduced sequence.

As used herein, the term "transfection" or "transformation" means the introduction of a nucleic acid into a cell. The introduced gene or sequence may be called a "clone". A host cell that receives the introduced DNA or RNA has been "transformed" and is a "transformant" or a "clone". The DNA or RNA introduced to a host cell may come from any source, including cells of the same genus or species as the host cell, or cells of a different genus or species.

As used herein, the term "host cell" means any cell of any organism that is selected, modified, transfected, transformed, grown, or used or manipulated in any way, for the production of a substance by the cell, for example the expression or replication, by the cell, of a gene, a DNA or RNA sequence, a protein or an enzyme.

As used herein, the term "expression system" means a host cell and compatible vector which, under suitable conditions, may express a protein or nucleic acid which is carried by the vector and introduced to the host cell. Common expression systems include *E. coli* host cells and plasmid vectors, insect host cells and Baculovirus vectors, and mammalian host cells and vectors. Suitable cells include CHO (chinese hamster ovary) cells, HeLa cells and NIH 3T3 cells and NSO cells (non-Ig-producing murine myeloma cell line). Nucleic acids encoding an antibody or antigen-binding fragment of the invention may be expressed at high levels in an *E.coli*/T7 expression system as disclosed in U.S. Patent Nos. 4,952,496; 5,693,489 and 5,869,320 and in Davanloo et al., (1984) Proc. Natl. Acad. Sci. USA 81:2035-2039; Studier et al., (1986) J. Mol. Biol. 189: 113-130; Rosenberg et al., (1987) Gene 56:125-135; and Dunn et al., (1988) Gene 68:259.

As used herein, the term "conservative substitution" refers to substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity *(see, e.g.,* Watson, et al., Molecular Biology of the Gene, The Benjamin/Cummings Pub. Co., p. 224 (4th Edition 1987)). Such exemplary substitutions are preferably made in accordance with those set forth in TABLE 1 as follows:

**TABLE 1**

| **Original residue** | **Conservative substitution** |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions are also permissible and may be determined empirically or in accord with known conservative substitutions.

As used herein, the term "isolated nucleic acid" or "isolated polypeptide" may refer to a nucleic acid, such as an RNA or DNA molecule or a mixed polymer, or to a polypeptide, respectively, which is partially or fully separated from other components that are normally found in cells or in recombinant DNA expression systems. These components include, but are not limited to, cell membranes, cell walls, ribosomes, polymerases, serum components, and flanking genomic sequences. The term thus includes a nucleic acid that has been removed from its naturally occurring environment, and may include recombinant or cloned DNA isolates and chemically synthesized analogs or analogs biologically synthesized by heterologous systems. An isolated nucleic acid or polypeptide will, preferably, be an essentially homogeneous composition of molecules but may contain some heterogeneity.

As used herein, the terms "polypeptide", "peptide" and "protein" encompass all such modifications, particularly those that are present in polypeptides synthesized by expressing a polynucleotide in a host cell.

As used herein, the term "antisense" refers to any composition containing nucleotide sequences which are complementary to a specific DNA or RNA sequence. The term "antisense strand" is used in reference to a nucleic acid strand that is complementary to the "sense" strand. Antisense molecules include peptide nucleic acids and may be produced by any method including synthesis or transcription. Once introduced into a cell, the complementary nucleotides combine with natural sequences produced by the cell to form duplexes and block either transcription or translation. The designation "negative" is sometimes used in reference to the antisense strand, and "positive" is sometimes used in reference to the sense strand.

As used herein, the term "antigenic determinant" refers to that fragment of a molecule (*i.e.,* an epitope) that makes contact with a particular antibody. When a protein or fragment of a protein is used to immunize a host animal, numerous regions of the protein may induce the production of antibodies which bind specifically to a given region or three-dimensional structure on the protein; these regions or structures are referred to as antigenic determinants. An antigenic determinant may compete with the intact antigen (*i.e.,* the immunogen used to elicit the immune response) for binding to an antibody.

As used herein, the term "antibody molecule" includes, but is not limited to, antibodies and fragments, preferably antigen-binding fragments, thereof. The term includes monoclonal antibodies, polyclonal antibodies, bispecific antibodies, Fab antibody fragments, F(ab)₂ antibody fragments, Fv antibody fragments (e.g., V_{H} or V_{L}), single chain Fv antibody fragments (scFv) and dsFv antibody fragments. Furthermore, the antibody molecules of the invention may be fully human antibodies or chimeric antibodies.

As used herein, the term "K_{off}" refers to the off-rate constant for dissociation of the antibody from an antibody/antigen complex.

As used herein, the term "Kₒₙ" refers to the rate at which the antibody associates with the antigen.

As used herein, the term "K_{d}" refers to the dissociation constant of a particular antibody/antigen interaction. K_{d} = K_{off}/Kₒₙ.

As used herein, the term "monoclonal antibody" refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Monoclonal antibodies are advantageous in that they may be synthesized by a hybridoma culture, essentially uncontaminated by other immunoglobulins. The modifier "monoclonal" indicates the character of the antibody as being amongst a substantially homogeneous population of antibodies, and is not to be construed as requiring production of the antibody by any particular method. As mentioned above, the monoclonal antibodies to be used in accordance with the present invention may be made by the hybridoma method first described by Kohler, et al., (1975) Nature 256: 495.

As used herein, the term "polyclonal antibody" refers to an antibody which was produced among or in the presence of one or more other, non-identical antibodies. In general, polyclonal antibodies are produced from a B-lymphocyte in the presence of several other B-lymphocytes which produced non-identical antibodies. Usually, polyclonal antibodies are obtained directly from an immunized animal.

As used herein, the term, "bispecific antibody" refers to an artificial hybrid antibody having two different heavy/light chain pairs and two different binding sites. Bispecific antibodies may be produced by a variety of methods including fusion of hybridomas or linking of Fab' fragments. See, *e.g.,* Songsivilai et al., (1990) Clin. Exp. Immunol. 79:315-321, Kostelny et al., (1992) J Immunol. 148:1547-1553. In addition, bispecific antibodies may be formed as "diabodies" (Holliger et al., (1993) PNAS USA 90:6444-6448) or as "Janusins" (Traunecker et al., (1991) EMBO J. 10:3655-3659 and Traunecker et al., (1992) Int. J. Cancer Suppl. 7:51-52).

As used herein, "bifunctional antibodies" or "immunocytokines" are antibody-cytokine fusion proteins comprising, in an amino-terminal to carboxy-terminal direction, (i) the antibody binding site comprising an immunoglobulin variable region capable of binding a cell surface antigen on the preselected cell-type, an immunoglobulin CH1 domain, an immunoglobulin CH2 domain (optionally a CH3 domain), and (ii) the cytokine. Methods of making such bifunctional immunocytokines are described in Gillies et al. (1992) Proc. Nat'l. Acad. Sci. 89: 1428-1432; Gillies et al. (1998) J. Immunol. 160:6195-6203; and U.S. Pat. No. 5,650,150.

As used herein, the term "anti-idiotypic antibodies" or "anti-idiotypes" refers to antibodies directed against the antigen-combining region or variable region (called the idiotype) of another antibody molecule. As disclosed by Jerne *et al.* (Jerne, N. K., (1974) Ann. Immunol. (Paris) 125c:373 and Jerne, N. K., et al., (1982) EMBO 1:234), immunization with an antibody molecule expressing a paratope (antigen-combining site) for a given antigen (e.g., an MDL-1 peptide) will produce a group of anti-antibodies, some of which share, with the antigen, a complementary structure to the paratope. Immunization with a subpopulation of the anti-idiotypic antibodies will, in turn, produce a subpopulation of antibodies or immune cell subsets that are reactive to the initial antigen.

As used herein, the term "fully human antibody" refers to an antibody which comprises human immunoglobulin protein sequences only. A fully human antibody may contain murine carbohydrate chains if produced in a mouse, in a mouse cell or in a hybridoma derived from a mouse cell. Similarly, "mouse antibody" refers to an antibody which comprises mouse immunoglobulin sequences only.

"Humanized" anti-MDL-1 peptide antibodies are also within the scope of the present invention. As used herein, the term "humanized" or "fully humanized" refers to an antibody that contains the amino acid sequences from the six complementarity-determining regions (CDRs) of the parent antibody, e.g., a mouse antibody, grafted to a human antibody framework. Humanized forms of non-human (*e.g.*, murine or chicken) antibodies are chimeric immunoglobulins, which contain minimal sequence derived from non-human immunoglobulin. For the most part, humanized antibodies are human immunoglobulins (recipient antibody) in which residues from a complementary determining region of the recipient are replaced by residues from a complementary determining region of a non-human species (donor antibody), such as mouse, chicken, rat or rabbit, having a desired specificity, affinity and capacity. In some instances, Fv framework residues of the human immunoglobulin are also replaced by corresponding non-human residues.

As used herein, the term "partially humanized" or "chimeric" antibody means an antibody that contains heavy and light chain variable regions of, e.g., murine origin, joined onto human heavy and light chain constant regions.

An alternative to humanization is to use human antibody libraries displayed on phage or human antibody libraries contained in transgenic mice, see, e.g., Vaughan et al. (1996) Nat. Biotechnol. 14:309-314; Barbas (1995) Nature Med. 1:837-839; de Haard et al. (1999) J. Biol. Chem. 274:18218-18230; McCafferty et al. (1990) Nature 348:552-554; Clackson et al. (1991) Nature 352:624-628; Marks et al. (1991) J. Mol. Biol. 222:581-597; Mendez et al. (1997) Nature Genet. 15:146-156; Hoogenboom and Chames (2000) Immunol. Today 21:371-377; Barbas et al. (2001) Phage Display: A Laboratory Manual, Cold Spring Harbor Laboratory Press, Cold Spring Harbor, New York; Kay et al. (1996) Phage Display of Peptides and Proteins: A Laboratory Manual, Academic Press, San Diego, CA; de Bruin et al. (1999) Nat. Biotechnol. 17:397-399.

As used herein, the term "human" refers to antibodies containing amino acid sequences that are of 100% human origin, where the antibodies may be expressed, e.g., in a human, animal, insect, fungal, plant, bacterial, or viral host *(*Baca et al. (1997) J. Biol. Chem. 272:10678-10684; Clark (2000) Immunol. Today 21:397-402).

The present invention includes "chimeric antibody" which means an antibody that comprises a variable region of the present invention fused or chimerized with an antibody region *(e.g.,* constant region) from another, non-human species *(e.g.,* mouse, horse, rabbit, dog, cow, chicken). These antibodies may be used to modulate the expression or activity of MDL-1 in the non-human species.

As used herein, the term "human/mouse chimeric antibody" refers to an antibody which comprises a mouse variable region (V_{H} and V_{L}) fused to a human constant region.

As used herein, the term "single-chain Fv" or "sFv" antibody fragments means antibody fragment that have the V_{H} and V_{L} domains of an antibody, wherein these domains are present in a single polypeptide chain. Generally, the sFv polypeptide further comprises a polypeptide linker between the V_{H} and V_{L} domains which enables the sFv to form the desired structure for antigen binding. Techniques described for the production of single chain antibodies (U.S. Patent Nos. 5,476,786, 5,132,405 and 4,946,778) may be adapted to produce anti-MDL-1-specific single chain antibodies. For a review of sFv see Pluckthun in The Pharmacology of Monoclonal Antibodies, vol. 113, Rosenburg and Moore eds. Springer-Verlag, N.Y., pp. 269-315 (1994).

Single chain antibodies, single domain antibodies, and bispecific antibodies are described, see, *e.g.,* Malecki et al. (2002) Proc. Natl. Acad. Sci. USA 99:213-218; Conrath et al. (2001) J. Biol. Chem. 276:7346-7350; Desmyter et al. (2001) J. Biol. Chem. 276:26285-26290, Kostelney et al. (1992) J. Immunol. 148:1547-1553; U.S. Pat. Nos. 5,932,448; 5,532,210; 6,129,914; 6,133,426; 4,946,778.

As used herein, the terms "disulfide stabilized Fv fragments" and "dsFv" refer to antibody molecules comprising a variable heavy chain (V_{H}) and a variable light chain (V_{L}) which are linked by a disulfide bridge.

An "effective amount" of a composition of the invention may be an amount that will ameliorate one or more of the well-known parameters that characterize medical conditions caused or mediated by the MDL-1 receptor or a functional fragment thereof. By "effective amount" it is also meant the amount or concentration of antibody needed to bind to the target antigens e.g., MDL-1, expressed on the infiltrating leukocytes to cause a reduction or prevention of inflammation, autoimmunity or reperfusion injury.

"Inflammation" or "inflammatory disorder" as used herein is an immunological response, e.g., leukocyte migration, to an injury or foreign agent that destroys not only the agent but surrounding tissues. Inflammation can occur, e.g., in the digestive, respiratory, reproductive, excretory, musculoskeletal, and nervous systems. Examples of inflammatory disorders, include, but are not limited to, inflammatory bowel disorder, Crohn's disease, pulmonary hyperreactivity, nephritis, arthritis (e.g., osteoarthritis), skin inflammation (e.g., psoriasis, atopic dermatitis), etc.

"Autoimmunity" or "autoimmune disorder" are conditions characterized by specific humoral (B cell) or cell-mediated (T cell) mediated immune responses against constituents of the body's own tissues (self antigens or autoantigens). Examples of autoimmunity include, but are not limited to, systemic lupus erythematosus, multiple sclerosis, insulin-dependent diabetes, Graves disease, Hashimoto's thyroiditis, Addison's disease, rheumatoid arthritis, Goodpasture syndrome, scleroderma, myasthenia gravis, pernicious anemia, etc.

As used herein, the term "bone disorder" refers to a disease characterized by bone loss, i.e., a disease, condition, disorder or syndrome that has as a symptom or pathology a decrease in bone mass or density. Examples of diseases characterized by bone loss include, but are not limited to, osteolysis, including osseous metastasis, aseptic prosthetic loosening, periodontitis, osteoporosis, Paget's disease, metastatic bone disease, and rheumatoid arthritis. Such bone disorders include those associated with autoimmune diseases such as lupus and rheumatoid arthritis. It has been found that women with SLE had significantly lower bone mineral density T-scores than women without elevated SLE disease damage regardless of prior corticosteroid use status. Such bone disorders also include conditions associated with low bone mass, including a condition where the level of bone mass is below the age specific normal as defined in standards by the World Health Organization "Assessment of Fracture Risk and its Application to Screening for Postmenopausal Osteoporosis (1994). Report of a World Health Organization Study Group. World Health Organization Technical Series 843". Included in condition(s) associated with low bone mass are primary and secondary osteoporosis.

Also included are periodontal disease, alveolar bone loss, post-osteotomy and childhood idiopathic bone loss, as well as long-term complications of osteoporosis such as curvature of the spine, loss of height, and prosthetic surgery. Such bone disorders may affect those who present with low bone mass, such as vertebrates, e.g., mammals, known to have a significantly higher than average chance of developing such diseases as are described above including osteoporosis (e.g., post-menopausal women, men over the age of 50). The disorder can be treated with bone-mass-augmenting or-enhancing methods, including bone restoration, increasing the bone fracture healing rate, replacing bone graft surgery entirely, enhancing the rate of successful bone grafts, bone healing following facial reconstruction or maxillary reconstruction or mandibular reconstruction, prosthetic ingrowth, vertebral synostosis or long bone extension. Those skilled in the art will recognize that the term bone mass actually refers to bone mass per unit area, which is sometimes (although not strictly correctly) referred to as bone mineral density.

Examples of bone disorders herein include osteoporosis, such as primary or secondary osteoporosis, and including glucocorticoid-induced osteoporosis, a focal bone erosion or disease such as that from rheumatoid arthritis and including marginal joint erosions and subchondral bone erosions (bone marrow), Paget's disease, a bone defect, abnormally increased bone turnover, periodontal disease, tooth loss, periprosthetic osteolysis, osteogenesis imperfecta, metastatic bone disease, hypercalcemia of malignancy, childhood idiopathic bone loss, alveolar bone loss, bone fracture, osteopenia such as juxta-articular osteopenia, bone disease in multiple myeloma and related conditions such as Waldenstroms' macroglobulinemia and/or monoclonal gammopathy. Preferred bone disorders herein are bone disease in multiple mycloma, macroglulinemia and monoclonal gammopathy and osteoporosis, more preferably secondary osteoporosis, and more preferably still bone loss during inflammation. Bone disorders that are not associated with a malignancy are also within the scope of the invention.

"Secondary osteoporosis" includes bone loss during inflammation, glucocorticoid-induced osteoporosis, hyperthyroidism-induced osteoporosis, immobilization-induced osteoporosis, heparin-induced osteoporosis and immunosuppressive-induced osteoporosis in a vertebrate, e.g., a mammal (including a human being). As used herein, the term "bone resorption" refers to the undesired loss of bone caused at least in part by osteoclast activity.

"Osteolysis" refers to catastrophic bone loss, or a debilitating pathological consequence of a spectrum of disease states including rheumatoid arthritis, osseous metastasis, aseptic prosthetic loosening and periodontitis. Rheumatoid arthritis (RA) is a chronic inflammatory disease which often results in long-term disability and increased mortality.

"Osteoprogenitor" refers to a differentiated bone precursor cell derived from a bone stromal cell.

"Odontoprogenitor" refers to a differentiated bone precursor cell derived from periodontal ligament.

### DETAILED DESCRIPTION OF THE INVENTION

The terms "MDL-1", "Myeloid DAP12 associating lectin-1", "Myeloid DAP12-associated lectin-1", DAP-12", "DAP12", "DNAX Activation Protein, 12 kD" are well known in the art. The human and mouse DAP 12 and MDL-1 nucleotide and polypeptide sequences are disclosed in WO 99/06557. The human MDL-1 nucleotide and amino acid sequences are defined by SEQ ID NO: 11 and SEQ ID NO: 12 of WO 99/06557, respectively. GenBank^{®} deposits of the human MDL-1 nucleic acid sequence (AR217548) and mouse MDL-1 nucleic and amino acid sequences (AR217549 and AAN21593, respectively) are also available.

A structural feature of the MDL-1 protein is the extracellular domain, which is defined by amino acid residues 26 to 188 of SEQ ID NO: 2 of a human MDL-1 protein, and amino acid residues 26 to 190 of SEQ ID NO: 4 of a mouse MDL-1 protein. Soluble MDL-1 protein can be fused to heterologous proteins, e.g., the Fc portion of antibody, or conjugated to chemical moieties, e.g., PEG.

Soluble MDL-1 polypeptides may be used as therapeutics or diagnostics similar to the use of MDL-1 antibodies or antigen-binding fragments thereof. The cell surface expression of MDL-1 indicates that this molecule is an attractive target for antibody-based therapeutic strategies. MDL-1 antibodies may be introduced into a patient such that the antibody binds to MDL-1

The present invention is based upon the discovery that MDL-1 exacerbates inflammatory bone destruction, while MDL-1 antagonists prevented this type of tissue destruction.

### Molecular Biology

In accordance with the present invention there may be employed conventional molecular biology, microbiology, and recombinant DNA techniques within the skill of the art. Such techniques are explained fully in the literature. Sec, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook, *et al.,* 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds. (1985)); Transcription And Translation (B.D. Hames & S.J. Higgins, cds. (1984)); Animal Cell Culture (R.I. Freshney, ed. (1986)); Immobilized Cells And Enzymes (IRL Press, (1986)); B. Perbal, A Practical Guide To Molecular Cloning (1984); F.M. Ausubel et al. (eds.), Current Protocols in Molecular Biology, John Wiley & Sons, Inc. (1994).

The present invention includes recombinant versions of the MDL-1 antibody or antigen-binding fragment of the invention.

Also disclosed is a nucleic acid, which encodes MDL-1, a soluble MDL-1, an anti-MDL-1 antibody, an anti-MDL-1 antibody heavy or light chain, an anti-MDL-1 antibody heavy or light chain variable region, an anti-MDL-1 antibody heavy or light chain constant region or anti-MDL-1 antibody CDR (e.g., CDR- L1, CDR-L2, CDR-L3, CDR-H1, CDR-H2 or CDR-H3), which may be amplified by PCR.

The sequence of any nucleic acid *(e.g.,* a nucleic acid encoding an MDL-1 gene or a nucleic acid encoding an anti-MDL-1 antibody or a fragment or portion thereof) may be sequenced by any method known in the art (e.g., chemical sequencing or enzymatic sequencing). "Chemical sequencing" of DNA may denote methods such as that of Maxam and Gilbert (1977) (Proc. Natl. Acad. Sci. USA 74:560), in which DNA is randomly cleaved using individual base-specific reactions. "Enzymatic sequencing" of DNA may denote methods such as that of Sanger (Sanger et al., (1977) Proc. Natl. Acad. Sci. USA 74:5463).

The nucleic acids herein may be flanked by natural regulatory (expression control) sequences, or may be associated with heterologous sequences, including promoters, internal ribosome entry sites (IRES) and other ribosome binding site sequences, enhancers, response elements, suppressors, signal sequences, polyadenylation sequences, introns, 5'-and 3'- non-coding regions, and the like.

Promoters, which may be used to control gene expression, include, but are not limited to, the cytomegalovirus (CMV) promoter (U.S. Patent Nos. 5,385,839 and 5,168,062), the SV40 early promoter region (Benoist et al., (1981) Nature 290:304-310), the promoter contained in the 3' long terminal repeat of Rous sarcoma virus (Yamamoto et al., (1980) Cell 22:787-797), the herpes thymidine kinase promoter (Wagner et al., (1981) Proc. Natl. Acad. Sci. USA 78:1441-1445), the regulatory sequences of the metallothionein gene (Brinster et al., (1982) Nature 296:39-42); prokaryotic expression vectors such as the β-lactamase promoter (Villa-Komaroff et al., (1978) Proc. Natl. Acad. Sci. USA 75:3727-3731), or the tac promoter (DeBoer et al., (1983) Proc. Natl. Acad. Sci. USA 80:21-25); see also "Useful proteins from recombinant bacteria" in Scientific American (1980) 242:74-94; and promoter elements from yeast or other fungi such as the Gal 4 promoter, the ADC (alcohol dehydrogenase) promoter, PGK (phosphoglycerol kinase) promoter or the alkaline phosphatase promoter.

A coding sequence is "under the control of", "functionally associated with" or "operably associated with" transcriptional and translational control sequences in a cell when the sequences direct RNA polymerase mediated transcription of the coding sequence into RNA, preferably mRNA, which then may be trans-RNA spliced (if it contains introns) and, optionally, translated into a protein encoded by the coding sequence.

Also included in the present disclosure are nucleic acids comprising nucleotide sequences and polypeptides comprising amino acid sequences that are at least 70% identical, at least 80% identical, at least 90% identical e.g., 91%, 92%, 93%, 94%, and at least 95% identical e.g., 95%, 96%, 97%, 98%, 99%, 100%, to the reference nucleotide and amino acid sequences of Table 1 when the comparison is performed by a BLAST algorithm wherein the parameters of the algorithm are selected to give the largest match between the respective sequences over the entire length of the respective reference sequences. Polypeptides comprising amino acid sequences which are at least 70% similar, at least 80% similar, at least 90% similar e.g., 91%, 92%, 93%, 94%, and at least 95% similar e.g., 95%, 96%, 97%, 98%, 99%, 100%, to the reference amino acid sequences of Table 1 *e.g.,* SEQ ID NOs: 2 and 4, when the comparison is performed with a BLAST algorithm wherein the parameters of the algorithm are selected to give the largest match between the respective sequences over the entire length of the respective reference sequences, are also included in the present disclosure.

Sequence identity refers to exact matches between the nucleotides or amino acids of two sequences which are being compared. Sequence similarity refers to both exact matches between the amino acids of two polypeptides which are being compared in addition to matches between nonidentical, biochemically related amino acids. Biochemically related amino acids which share similar properties and may be interchangeable are discussed above.

Soluble MDL-1 protein comprises the extracellular domain of MDL-1. Moreover, fragments of the extracellular domain will also provide soluble forms of the MDL-1 protein. Fragments can be prepared using known techniques to isolate a desired portion of the extracellular region.

Conventional molecular biology techniques can be used to produce chimeric proteins having MDL-1 fused a heterologous enzymatically inactive polypeptide (e.g., a lytic or non-lytic Fc region of IgG). Preferably, the protein has a molecular weight of at least 10 kD; a net neutral charge at pH 6.8; a globular tertiary structure; and of human origin. Where the enzymatically inactive polypeptide is IgG, preferably, the IgG portion is glycosylated. If desired, the enzymatically inactive polypeptide can include an IgG hinge region positioned such that the chimeric protein has MDL-1 bonded to an IgG hinge region with the hinge region bonded to a longevity-increasing polypeptide. Thus, the hinge region can serve as a spacer between the cytokine and the longevity-increasing polypeptide. A person skilled in molecular biology can readily produce such molecules from an IgG2a-secreting hybridoma (e.g., HB129) or other eukaryotic cells or baculovirus systems. As an alternative to using an IgG hinge region, a flexible polypeptide spacer, as defined herein, can be used. Using conventional molecular biology techniques, such a polypeptide can be inserted between MDL-1 and the longevity-increasing polypeptide.

Where the heterologous protein includes an Fc region, the Fc region can be mutated, if desired, to inhibit its ability to fix complement and bind the Fc receptor with high affinity. For murine IgG Fc, substitution of Ala residues for Glu 318, Lys 320, and Lys 322 renders the protein unable to direct ADCC. Substitution of Glu for Leu 235 inhibits the ability of the protein to bind the Fc receptor with high affinity. Appropriate mutations for human IgG also are known (see, e.g., Morrison et al., 1994, The Immunologist 2: 119-124 and Brekke et al., 1994, The Immunologist 2: 125). Other mutations can also be used to inhibit these activities of the protein, and art-recognized methods can be used to assay for the ability of the protein to fix complement or bind the Fc receptor. Other useful heterologous polypeptides include albumin (e.g., human serum albumin), transferrin, enzymes such as t-PA which have been inactivated by mutations, and other proteins with a long circulating half-life and without enzymatic activity in humans.

The chimeric proteins can be synthesized (e.g., in mammalian cells) using conventional methods for protein expression using recombinant DNA technology. Because many of the polypeptides used to create the chimeric proteins have been previously purified, many of the previously-described methods of protein purification should be useful, along with other conventional methods, for purifying the chimeric proteins. If desired, the chimeric protein can be affinity-purified according to standard protocols with antibodies directed against the cytokine. Antibodies directed against the enzymatically inactive protein are also useful for purifying the chimeric protein by conventional immunoaffinity techniques. If desired, the activity of the chimeric protein can be assayed with methods that are commonly used to test the activity of the protein alone. It is not necessary that the activity of the chimeric protein be identical to the activity of the protein alone.

The present disclosure also includes fusions which include the polypeptides and polynucleotides of the present invention and a second polypeptide or polynucleotide moiety, which may be referred to as a "tag". The fused polypeptides of the invention may be conveniently constructed, for example, by insertion of a polynucleotide of the invention or fragment thereof into an expression vector as described above. The fusions of the invention may include tags which facilitate purification or detection. Such tags include glutathione-S-transferase (GST), hexahistidine (His6) tags, maltose binding protein (MBP) tags, haemagglutinin (HA) tags, cellulose binding protein (CBP) tags and myc tags. Detectable labels or tags such as ³²P, ³⁵S, ¹⁴C, ³H, ^{99m}Tc, ¹¹¹In, ⁶⁸Ga, ¹⁸F, ¹²⁵I, ¹³¹I, ^{113m}In, ⁷⁶Br, ⁶⁷Ga, ^{99m}Tc, ¹²³I, ¹¹¹In and ⁶⁸Ga may also be used to label the polypeptides of the invention. Methods for constructing and using such fusions are very conventional and well known in the art.

Modifications (e.g., post-translational modifications) that occur in a polypeptide often will be a function of how it is made. For polypeptides made by expressing a cloned gene in a host, for instance, the nature and extent of the modifications, in large part, will be determined by the host cell's post-translational modification capacity and the modification signals present in the polypeptide amino acid sequence. For instance, as is well known, glycosylation often does not occur in bacterial hosts such as *E. coli.* Accordingly, when glycosylation is desired, a polypeptide may be expressed in a glycosylating host, generally a eukaryotic cell. Insect cells often carry out post-translational glycosylations which are similar to those of mammalian cells. For this reason, insect cell expression systems have been developed to express, efficiently, mammalian proteins having native patterns of glycosylation. Alternatively, deglycosylation enzymes may be used to remove carbohydrates attached during production in eukaryotic expression systems.

Analogs of the MDL-1 peptides may be prepared by chemical synthesis or by using site-directed mutagenesis, Gillman et al., (1979) Gene 8:81; Roberts et al., (1987) Nature, 328:731 or Innis (Ed.), 1990, PCR Protocols: A Guide to Methods and Applications, Academic Press, New York, NY or the polymerase chain reaction method PCR; Saiki et al., (1988) Science 239:487, as exemplified by Daugherty et al., (1991) (Nucleic Acids Res. 19:2471) to modify nucleic acids encoding the peptides. Adding epitope tags for purification or detection of recombinant products is envisioned.

Still other analogs are prepared by the use of agents known in the art for their usefulness in cross-linking proteins through reactive side groups. Preferred derivatization sites with cross-linking agents are free amino or carboxy groups, carbohydrate moieties and cysteine residues.

### Protein Purification

Typically, the peptides of the invention may be produced by expressing a nucleic acid which encodes the polypeptide in a host cell which is grown in a culture (e.g., liquid culture such as Luria broth). For example, the nucleic acid may be part of a vector (e.g., a plasmid) which is present in the host cell. Following expression, the peptides of the invention may be isolated from the cultured cells. The peptides of this invention may be purified by standard methods, including, but not limited to, salt or alcohol precipitation, affinity chromatography (e.g., used in conjunction with a purification tagged peptide as discussed above), preparative disc-gel electrophoresis, isoelectric focusing, high pressure liquid chromatography (HPLC), reversed-phase HPLC, gel filtration, cation and anion exchange and partition chromatography, and countercurrent distribution. Such purification methods are very well known in the art and are disclosed, e.g., in "Guide to Protein Purification", Methods in Enzymology, Vol. 182, M. Deutscher, Ed., 1990, Academic Press, New York, NY.

### Antibody Structure

In general, the basic antibody structural unit is known to comprise a tetramer. Each tetramer includes two identical pairs of polypeptide chains, each pair having one "light" (about 25 kDa) and one "heavy" chain (about 50-70 kDa). The amino-terminal portion of each chain may include a variable region of about 100 to 110 or more amino acids primarily responsible for antigen recognition. The carboxy-terminal portion of each chain may define a constant region primarily responsible for effector function. Typically, human light chains are classified as kappa and lambda light chains. Furthermore, human heavy chains are typically classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)) (incorporated by reference in its entirety for all purposes).

The variable regions of each light/heavy chain pair may form the antibody binding site. Thus, in general, an intact IgG antibody has two binding sites. Except in bifunctional or bispecific antibodies, the two binding sites are, in general, the same.

Normally, the chains all exhibit the same general structure of relatively conserved framework regions (FR) joined by three hypervariable regions, also called complementarity determining regions or CDRs. The CDRs from the two chains of each pair are usually aligned by the framework regions, enabling binding to a specific epitope. In general, from N-terminal to C-terminal, both light and heavy chains comprise the domains FR1, CDR1, FR2, CDR2, FR3, CDR3 and FR4. The assignment of amino acids to each domain is, generally, in accordance with the definitions of Sequences of Proteins of Immunological Interest, Kabat et al.; National Institutes of Health, Bethesda, Md. ; 5th ed.; NIH Publ. No. 91-3242 (1991); Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 342:878-883.

### Antibody Molecules

The anti-MDL-1 antibody molecules of the invention preferably recognize human MDL-1. For example, the polypeptide expressed by the genes comprising the polynucleotide sequence of SEQ ID NO: 1. For example, the soluble MDL-1 polypeptide which is defined by amino acid residues 26 to 188 of SEQ ID NO: 2 of a human MDL-1 protein. However, the present invention includes antibody molecules which recognize mouse MDL-1, and MDL-1 from other species, preferably mammals (e.g., rat, rabbit, sheep or dog). For example, the polypeptide expressed by the genes comprising the polynucleotide sequence of SEQ ID NO: 3. For example, the soluble MDL-1 polypeptide which is defined by amino acid residues 26 to 190 of SEQ ID NO: 4 of a murine MDL-1 protein. The present invention also includes anti-MDL-1 antibodies or fragments thereof which are complexed with MDL-1 or any fragment thereof or with any cell which is expressing MDL-1 or any portion or fragment thereof on the cell surface. Such complexes may be made by contacting the antibody or antibody fragment with MDL-1 or the MDL-1 fragment.

In an embodiment, fully-human monoclonal antibodies directed against MDL-1 are generated using transgenic mice carrying parts of the human immune system rather than the mouse system. These transgenic mice, which may be referred to, herein, as "HuMAb" mice, contain a human immunoglobulin gene miniloci that encodes unrearranged human heavy (µ and γ) and κ light chain immunoglobulin sequences, together with targeted mutations that inactivate the endogenous µ and κ chain loci (Lonberg, N., et al., (1994) Nature 368(6474):856-859). These antibodies are also referred to as fully human antibodies. Accordingly, the mice exhibit reduced expression of mouse IgM or κ, and in response to immunization, the introduced human heavy and light chain transgenes undergo class switching and somatic mutation to generate high affinity human IgGκ monoclonal antibodies (Lonberg, *N., et al.,* (1994), *supra*; reviewed in Lonberg, N. (1994) Handbook of Experimental Pharmacology 113:49-101; Lonberg et al., (1995) Intern.Rev. Immunol. 13:65-93, and Harding et al., (1995) Ann. N. Y Acad. Sci 764:536-546). The preparation of HuMab mice is commonly known in the art and is described, for example, in Taylor et al., (1992) Nucleic Acids Research 20:6287-6295; Chen et al., (1993) International Immunology 5:647-656; Tuaillon et al., (1993) Proc. Natl. Acad. Sci USA 90:3720-3724; Choi et al., (1993) Nature Genetics 4:117-123; Chen et al., (1993) EMBO J. 12:821- 830; Tuaillon et al., (1994) J Immunol. 152:2912-2920; Lonberg et al., (1994) Nature 368(6474):856-859; Lonberg, N. (1994) Handbook of Experimental Pharmacology 113:49-101; Taylor et al., (1994) International Immunology 6:579-591; Lonberg et al., (1995) Intern. Rev. Immunol. Vol. 13:65-93; Harding et al., (1995) Ann. N.Y Acad. Sci 764:536-546; Fishwild et al., (1996) Nature Biotechnology 14:845-851 and Harding et al., (1995) Annals NY Acad. Sci. 764:536-546; the contents of all of which are hereby incorporated by reference in their entirety. See further, U.S. Patent Nos. 5,545,806; 5, 569,825; 5,625,126; 5,633,425; 5,789,650; 5,877,397; 5,661,016; 5,814,318; 5,874, 299; 5,770,429 and 5,545,807; and International Patent Application Publication Nos. WO 98/24884; WO 94/25585; WO 93/12227; WO 92/22645 and WO 92/03918. .

To generate fully human, monoclonal antibodies to MDL-1, HuMab mice may be immunized with an antigenic MDL-1 polypeptide as described by Lonberg et al., (1994) Nature 368(6474):856-859; Fishwild et al., (1996) Nature Biotechnology 14:845-851 and WO 98/24884. Preferably, the mice will be 6-16 weeks of age upon the first immunization. For example, a purified preparation of MDL-1 may be used to immunize the HuMab mice intraperitoneally. The mice may also be immunized with whole cells which are stably transformed or transfected with an MDL-1 gene.

In general, HuMAb transgenic mice respond well when initially immunized intraperitoneally (IP) with antigen in complete Freund's adjuvant, followed by every other week IP immunizations (usually, up to a total of 6) with antigen in incomplete Freund's adjuvant. Mice may be immunized, first, with cells expressing MDL-1, then with a soluble fragment of MDL-1 and continually receive alternating immunizations with the two antigens. The immune response may be monitored over the course of the immunization protocol with plasma samples being obtained by retroorbital bleeds. The plasma may be screened for the presence of anti-MDL-1 antibodies, for example by ELISA, and mice with sufficient titers of immunoglobulin may be used for fusions. Mice may be boosted intravenously with antigen 3 days before sacrifice and removal of the spleen. It is expected that 2-3 fusions for each antigen may need to be performed. Several mice may be immunized for each antigen. For example, a total of twelve HuMAb mice of the HC07 and HC012 strains may be immunized.

Hybridoma cells which produce the monoclonal anti-MDL-1 antibodies may be produced by methods which are commonly known in the art. These methods include, but are not limited to, the hybridoma technique originally developed by Kohler, et al., (1975) (Nature 256:495-497), as well as the trioma technique (Hering et al., (1988) Biomed. Biochim. Acta. 47:211-216 and Hagiwara et al., (1993) Hum. Antibod. Hybridomas 4:15), the human B-cell hybridoma technique (Kozbor et al., (1983) Immunology Today 4:72 and Cote et al., (1983) Proc. Natl. Acad. Sci. U.S.A 80:2026-2030), and the EBV-hybridoma technique (Cole et al., in Monoclonal Antibodies and Cancer Therapy, Alan R. Liss, Inc., pp. 77-96, 1985). Preferably, mouse splenocytes are isolated and fused with PEG to a mouse myeloma cell line based upon standard protocols. The resulting hybridomas may then be screened for the production of antigen-specific antibodies. For example, single cell suspensions of splenic lymphocytes from immunized mice may by fused to one-sixth the number of P3X63- Ag8.653 nonsecreting mouse myeloma cells (ATCC, CRL 1580) with 50% PEG. Cells may be plated at approximately 2 x 10⁵ cells/mL in a flat bottom microtiter plate, followed by a two week incubation in selective medium containing 20% fetal Clone Serum, 18% "653" conditioned media, 5% origen (IGEN), 4 mM L-glutamine, 1 mM L-glutamine, 1 mM sodium pyruvate, 5mM HEPES, 0.055 mM 2-mercaptoethanol, 50 units/ml penicillin, 50 mg/ml streptomycin, 50 mg/ml gentamycin and IX HAT (Sigma; the HAT is added 24 hours after the fusion). After two weeks, cells may be cultured in medium in which the HAT is replaced with HT. Individual wells may then be screened by ELISA for human anti-MDL-1 monoclonal IgG antibodies. Once extensive hybridoma growth occurs, medium may be observed usually after 10-14 days. The antibody secreting hybridomas may be replated, screened again, and if still positive for human IgG, anti-MDL-1 monoclonal antibodies, may be subcloned at least twice by limiting dilution. The stable subclones may then be cultured *in vitro* to generate small amounts of antibody in tissue culture medium for characterization.

The anti-MDL-1 antibody molecules of the present invention may also be produced recombinantly *(e.g.,* in an *E.coli*/T7 expression system as discussed above). In this embodiment, nucleic acids encoding the antibody molecules of the invention (e.g., V_{H} or V_{L}) may be inserted into a pET-based plasmid and expressed in the *E.coli*/T7 system. There are several methods by which to produce recombinant antibodies which are known in the art. One example of a method for recombinant production of antibodies is disclosed in U.S. Patent No. 4,816,567 which is herein incorporated by reference. Transformation may be by any known method for introducing polynucleotides into a host cell. Methods for introduction of heterologous polynucleotides into mammalian cells are well known in the art and include dextran-mediated transfection, calcium phosphate precipitation, polybrene-mediated transfection, protoplast fusion, electroporation, encapsulation of the polynucleotide(s) in liposomes, biolistic injection and direct microinjection of the DNA into nuclei. In addition, nucleic acid molecules may be introduced into mammalian cells by viral vectors. Methods of transforming cells are well known in the art. See, for example, U.S. Patent Nos. 4,399,216; 4,912,040; 4,740,461 and 4,959,455.

Mammalian cell lines available as hosts for expression are well known in the art and include many immortalized cell lines available from the American Type Culture Collection (ATCC). These include, *inter alia,* Chinese hamster ovary (CHO) cells, NSO, SP2 cells, HeLa cells, baby hamster kidney (BHK) cells, monkey kidney cells (COS), human hepatocellular carcinoma cells *(e.g.,* Hep G2), A549 cells, 3T3 cells, and a number of other cell lines. Mammalian host cells include human, mouse, rat, dog, monkey, pig, goat, bovine, horse and.hamster cells. Cell lines of particular preference are selected through determining which cell lines have high expression levels. Other cell lines that may be used are insect cell lines, such as Sf9 cells, amphibian cells, bacterial cells, plant cells and fungal cells. When recombinant expression vectors encoding the heavy chain or antigen-binding fragment thereof, the light chain and/or antigen-binding fragment thereof are introduced into mammalian host cells, the antibodies are produced by culturing the host cells for a period of time sufficient to allow for expression of the antibody in the host cells or, more preferably, 5 secretion of the antibody into the culture medium in which the host cells are grown.

Antibodies may be recovered from the culture medium using standard protein purification methods. Further, expression of antibodies of the invention (or other moieties therefrom) from production cell lines may be enhanced using a number of known techniques. For example, the glutamine synthetase gene expression system (the GS system) is a common approach for enhancing expression under certain conditions. The GS system is discussed in whole or part in connection with European Patent Nos. 0 216 846, 0 256 055, and 0 323 997 and European Patent Application No. 89303964.4.

It is likely that antibodies expressed by different cell lines or in transgenic animals will have different glycosylation from each other. However, all antibodies encoded by the nucleic acid molecules provided herein, or comprising the amino acid sequences provided herein are part of the instant invention, regardless of the glycosylation of the antibodies.

Antibody fragments, preferably antigen-binding antibody fragments, fall within the scope of the present invention also include F(ab)₂ fragments which may be produced by enzymatic cleavage of an IgG by, for example, pepsin. Fab fragments may be produced by, for example, reduction of F(ab)₂ with dithiothreitol or mercaptoethylamine. A Fab fragment is a V_{L}-C_{L} chain appended to a V_{H}-C_{H1} chain by a disulfide bridge. A F(ab)₂ fragment is two Fab fragments which, in turn, are appended by two disulfide bridges. The Fab portion of an F(ab)₂ molecule includes a portion of the F_{c} region between which disulfide bridges are located.

As is well known, Fv, the minimum antibody fragment which contains a complete antigen recognition and binding site, consists of a dimer of one heavy and one light chain variable domain (V_{H} -V_{L}) in non-covalent association. In this configuration that corresponds to the one found in native antibodies the three complementarity determining regions (CDRs) of each variable domain interact to define an antigen binding site on the surface of the V_{H} -V_{L} dimer. Collectively, the six CDRs confer antigen binding specificity to the antibody. Frameworks (FRs) flanking the CDRs have a tertiary structure that is essentially conserved in native immunoglobulins of species as diverse as human and mouse. These FRs serve to hold the CDRs in their appropriate orientation. The constant domains are not required for binding function, but may aid in stabilizing V_{H} -V_{L} interaction. Even a single variable domain (or half of an Fv comprising only three CDRs specific for an antigen) has the ability to recognize and bind antigen, although usually at a lower affinity than an entire binding site (Painter, Biochem. 11 (1972), 1327-1337). Hence, said domain of the binding site of the antibody construct as defined and described in the present invention may be a pair of V_{H} -V_{L}, V_{H} - V_{H} or V_{L} - V_{L} domains of different immunoglobulins. The order of V_{H} and V_{L} domains within the polypeptide chain is not decisive for the present invention, the order of domains given hereinabove may be reversed usually without any loss of function. It is important, however, that the V_{H} and V_{L} domains are arranged so that the antigen binding site may properly fold. An Fv fragment is a V_{L} or V_{H} region.

Depending on the amino acid sequences of the constant domain of their heavy chains, immunoglobulins may be assigned to different classes. There are at least five major classes of immunoglobulins: IgA, IgD, IgE, IgG and IgM, and several of these may be further divided into subclasses (isotypes), e.g. IgG-1, IgG-2, IgG-3 and IgG-4; IgA-1 and IgA-2.

The anti-MDL-1 antibody molecules of the invention may also be conjugated to a chemical moiety. The chemical moiety may be, *inter alia,* a polymer, a radionuclide or a cytotoxic factor. Preferably the chemical moiety is a polymer which increases the half-life of the antibody molecule in the body of a subject. Suitable polymers include, but are not limited to, polyethylene glycol (PEG) (e.g., PEG with a molecular weight of 2kDa, 5 kDa, 10 kDa, 12kDa, 20 kDa, 30kDa or 40kDa), dextran and monomethoxypolyethylene glycol (mPEG). Lee et al., (1999) (Bioconj. Chem. 10:973-981) discloses PEG conjugated single-chain antibodies. Wen et al., (2001) (Bioconj. Chem. 12:545-553) disclose conjugating antibodies with PEG which is attached to a radiometal chelator (diethylenctriaminpentaacetic acid (DTPA)).

The antibodies and antibody fragments of the invention may also be conjugated with labels such as ⁹⁹Tc, ⁹⁰Y, ¹¹¹In, ³²P, ¹⁴C, ¹²⁵I, ³H, ¹³¹I, ¹¹C, ¹⁵O, ¹³N, ¹⁸F, ³⁵S, ⁵¹Cr, ⁵⁷To, ²²⁶Ra, ⁶⁰Co, ⁵⁹Fe, ⁵⁷Se, ¹⁵²Eu, ⁶⁷CU, ²¹⁷Ci, ²¹¹At, ²¹²Pb, ⁴⁷Sc, ¹⁰⁹Pd, ²³⁴Th, and ⁴⁰K, ¹⁵⁷Gd, ⁵⁵Mn, ⁵²Tr and ⁵⁶Fe.

The antibodies and antibody fragments of the invention may also be conjugated with fluorescent or chemilluminescent labels, including fluorophores such as rare earth chelates, fluorescein and its derivatives, rhodamine and its derivatives, isothiocyanate, phycoerythrin, phycocyanin, allophycocyanin, o-phthaladehyde, fluorescamine, ¹⁵²Eu, dansyl, umbelliferone, lucifcrin, luminal label, isoluminal label, an aromatic acridinium ester label, an imidazole label, an acridimium salt label, an oxalate ester label, an aequorin label, 2,3-dihydrophthalazinediones, biotin/avidin, spin labels and stable free radicals.

The antibody molecules may also be conjugated to a cytotoxic factor such as diptheria toxin, *Pseudomonas aeruginosa* exotoxin A chain, ricin A chain, abrin A chain, modeccin A chain, alpha-sarcin, *Aleurites fordii* proteins and compounds (*e.g.,* fatty acids), dianthin proteins, *Phytoiacca american* proteins PAPI, PAPH, and PAP-S, *momordica charantia* inhibitor, curcin, crotin, *saponaria officinalis* inhibitor, mitogellin, restrictocin, phenomycin, and enomycin.

Any method known in the art for conjugating the antibody molecules of the invention to the various moieties may be employed, including those methods described by Hunter et al., (1962) Nature 144:945; David et al., (1974) Biochemistry 13:1014; Pain et al., (1981) J. Immunol. Meth. 40:219; and Nygren, J., (1982) Histochem. and Cytochem. 30:407. Methods for conjugating antibodies are conventional and very well known in the art.

Antigenic (*i.e.,* immunogenic) fragments of the MDL-1 peptides of the invention are within the scope of the present invention. Antigenic fragments may be joined to other materials, such as fused or covalently joined polypeptides, to be used as immunogens. The antigenic peptides may be useful for preparing antibody molecules which recognize MDL-1 or any fragment thereof. An antigen and its fragments may be fused or covalently linked to a variety of immunogens, such as keyhole limpet hemocyanin, bovine serum albumin, or ovalbumin (Coligan et al. (1994) Current Protocols in Immunol., Vol. 2, 9.3-9.4, John Wiley and Sons, New York, NY). Peptides of suitable antigenicity may be selected from the polypeptide target, using an algorithm, see, *e.g.,* Parker et al. (1986) Biochemistry 25:5425-5432; Jameson and Wolf (1988) Cabios 4:181-186; Hopp and Woods (1983) Mol. Immunol. 20:483-489.

Although it is not always necessary, when MDL-1 peptides are used as antigens to elicit antibody production in an immunologically competent host, smaller antigenic fragments are preferably first rendered more immunogenic by cross-linking or concatenation, or by coupling to an immunogenic carrier molecule (*i.e.,* a macromolecule having the property of independently eliciting an immunological response in a host animal, such as diptheria toxin or tetanus). Cross-linking or conjugation to a carrier molecule may be required because small polypeptide fragments sometimes act as haptens (molecules which are capable of specifically binding to an antibody but incapable of eliciting antibody production, *i.e.,* they are not immunogenic). Conjugation of such fragments to an immunogenic carrier molecule renders them more immunogenic through what is commonly known as the "carrier effect".

Carrier molecules include, e.g., proteins and natural or synthetic polymeric compounds such as polypeptides, polysaccharides, lipopolysaccharides, *etc.* Protein carrier molecules are especially preferred, including, but not limited to, keyhole limpet hemocyanin and mammalian serum proteins such as human or bovine gammaglobulin, human, bovine or rabbit serum albumin, or methylated or other derivatives of such proteins. Other protein carriers will be apparent to those skilled in the art. Preferably, the protein carrier will be foreign to the host animal in which antibodies against the fragments are to be elicited.

Covalent coupling to the carrier molecule may be achieved using methods well known in the art; the exact choice of which will be dictated by the nature of the carrier molecule used. When the immunogenic carrier molecule is a protein, the fragments of the invention may be coupled, *e.g.,* using water-soluble carbodiimides such as dicyclohexylcarbodiimide or glutaraldehyde.

Coupling agents, such as these, may also be used to cross-link the fragments to themselves without the use of a separate carrier molecule. Such cross-linking into aggregates may also increase immunogenicity. Immunogenicity may also be increased by the use of known adjuvants, alone or in combination with coupling or aggregation.

Adjuvants for the vaccination of animals include, but are not limited to, Adjuvant 65 (containing peanut oil, mannide monooleate and aluminum monostearate); Freund's complete or incomplete adjuvant; mineral gels such as aluminum hydroxide, aluminum phosphate and alum; surfactants such as hexadecylamine, octadecylamine, lysolecithin, dimethyldioctadecylammonium bromide, N,N-dioctadecyl-N',N'-bis(2-hydroxymethyl) propanediamine, methoxyhexadecylglycerol and pluronic polyols; polyanions such as pyran, dextran sulfate, poly IC, polyacrylic acid and carbopol; peptides such as muramyl dipeptide, dimethylglycine and tuftsin; and oil emulsions. The polypeptides could also be administered following incorporation into liposomes or other microcarriers.

Information concerning adjuvants and various aspects of immunoassays are disclosed, *e.g.,* in the series by P. Tijssen,_Practice and Theory of Enzyme Immunoassays, 3rd Edition, 1987, Elsevier, New York. Other useful references covering methods for preparing polyclonal antisera include Microbiology, 1969, Hoeber Medical Division, Harper and Row; Landsteiner, Specificity of Serological Reactions, 1962, Dover Publications, New York, and Williams, et al., Methods in Immunology and Immunochemistry, Vol. 1, 1967, Academic Press, New York.

The anti-MDL-1 "antibody molecules" of the invention include, but are by no means not limited to, anti-MDL-1 antibodies (*e.g.,* monoclonal antibodies, polyclonal antibodies, bispecific antibodies and anti-idiotypic antibodies) and fragments, preferably antigen-binding fragments, thereof, such as Fab antibody fragments, F(ab)₂ antibody fragments, Fv antibody fragments (*e.g.,* V_{H} or V_{L}), single chain Fv antibody fragments and dsFv antibody fragments. Furthermore, the antibody molecules of the invention may be fully human antibodies, mouse antibodies, rabbit antibodies, chicken antibodies, human/mouse chimeric antibodies or humanized antibodies.

The anti-MDL-1 antibody molecules of the invention preferably recognize human or mouse MDL-1 peptides of the invention; however, the present invention includes antibody molecules which recognize MDL-1 peptides from different species, preferably mammals (*e.g.,* pig, rat, rabbit, sheep or dog).

The present invention also includes complexes comprising the MDL-1 peptides of the invention and one or more antibody molecules, e.g., bifunctional antibodies. Such complexes may be made by simply contacting the antibody molecule with its cognate peptide.

Various methods may be used to make the antibody molecules of the invention. In preferred embodiments, the antibodies of the invention are produced by methods which are similar to those disclosed in U.S. Patent Nos. 5,625,126; 5,877,397; 6,255,458; 6,023,010 and 5,874,299. Hybridoma cells which produce monoclonal, fully human anti-MDL-1 peptide antibodies may then be produced by methods which are commonly known in the art. These methods include, but are not limited to, the hybridoma technique originally developed by Kohler et al., (1975) (Nature 256:495-497), as well as the trioma technique (Hering et al., (1988) Biomed. Biochim. Acta. 47:211-216 and Hagiwara et al., (1993) Hum. Antibod. Hybridomas 4:15), the human B-cell hybridoma technique (Kozbor et al., (1983) Immunology Today 4:72 and Cote et al., (1983) Proc. Natl. Acad. Sci. U.S.A. 80:2026-2030), and the EBV-hybridoma technique (Cole et al., in Monoclonal Antibodies and Cancer Therapy, Alan R. Liss, Inc., pp. 77-96, 1985). Again, ELISA may be used to determine if hybridoma cells are expressing anti-MDL-1 peptide antibodies.

Purification of antigen is not necessary for the generation of antibodies. Immunization may be performed by DNA vector immunization, see, e.g., Wang, et al. (1997) Virology 228:278-284. Alternatively, animals may be immunized with cells bearing the antigen of interest. Splenocytes may then be isolated from the immunized animals, and the splenocytes may be fused with a myeloma cell line to produce a hybridoma (Meyaard et al. (1997) Immunity 7:283-290; Wright et al. (2000) Immunity 13:233-242; Preston et al. (1997) Eur. J. Immunol. 27:1911-1918). Resultant hybridomas may be screened for production of the desired antibody by functional assays or biological assays, that is, assays not dependent on possession of the purified antigen. Immunization with cells may prove superior for antibody generation than immunization with purified antigen (Kaithamana et al. (1999) J. Immunol. 163:5157-5164).

Antibody to antigen and ligand to receptor binding properties may be measured, e.g., by surface plasmon resonance (Karlsson et al. (1991) J. Immunol. Methods 145:229-240; Neri et al. (1997) Nat. Biotechnol. 15:1271-1275; Jonsson et al. (1991) Biotechniques 11:620-627) or by competition ELISA (Friguet et al. (1985) J. Immunol. Methods 77:305-319; Hubble (1997) Immunol. Today 18:305-306). Antibodies may be used for affinity purification to isolate the antibody's target antigen and associated bound proteins, see, *e.g.,* Wilchek et al. (1984) Meth. Enzymol. 104:3-55.

Antibodies that specifically bind to variants of MDL-1, where the variant has substantially the same nucleic acid and amino acid sequence as those recited herein, but possessing substitutions that do not substantially affect the functional aspects of the nucleic acid or amino acid sequence, are within the definition of the contemplated methods. Variants with truncations, deletions, additions, and substitutions of regions which do not substantially change the biological functions of these nucleic acids and polypeptides are within the definition of the contemplated methods.

Bispecific antibodies are antibodies that have binding specificities for at least two different epitopes. Exemplary bispecific antibodies may bind to two different epitopes of MDL-1. Alternatively, bispecific MDL-1 antibodies can bind to another antigen, e.g., DC-SIGN, CD20, RANK-L, etc.

Methods for making bispecific antibodies are known in the art. Traditional production of full-length bispecific antibodies is based on the co-expression of two immunoglobulin heavy-chain-light-chain pairs, where the two chains have different specificities (Millstein et al. Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al. EMBO J, 10:3655-3659 (1991).

According to a different approach, antibody variable domains with the desired binding specificities (antibody-antigen combining sites) are fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding, present in at least one of the fusions. DNAs encoding the immunoglobulin heavy-chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy-chain-light-chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al. Methods in Enzymology, 121:210 (1986).

According to another approach described in U.S. Pat. No. 5, 731,168, the interface between a pair of antibody molecules can be engineered to maximize the percentage of heterodimers that are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al. Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al. (1992) J. Exp. Med., 175:217-225 describe the production of a fully humanized bispecific antibody F(ab')₂ molecule. Each Fab' fragment was separately secreted from *E. coli* and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al. (1992) J. Immunol., 148(5):1547-1553. The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al. (1993) Pro. Natl. Acad. Sci. USA, 90:6444-6448 has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al. (1994) J. Immunol., 152:5368.

Antibodies with more than two valencies are contemplated. For example, trispecific antibodies can be prepared. Tutt et al. (1991) J. Immunol. 147: 60.

### Antibody Binding Assays

The antibodies of the invention may be assayed for immunospecific binding by any method known in the art. The immunoassays which may be used include, but are not limited to, competitive and non-competitive assay systems using techniques, such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, *e.g.,* Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer, such as RIPA buffer (1% NP-40 or Triton X-100, 1% sodium deoxycholate, 0.1 % SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody of interest to immunoprecipitate a particular antigen may be assessed by, *e.g.,* western blot analysis. One of skill in the art would be knowledgeable as to the parameters that may be modified to increase the binding of the antibody to an antigen and decrease the background (*e.g.,* pre-clearing the cell lysate with sepharose beads). For further discussion regarding immunoprecipitation protocols see, *e.g.,* Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (*e.g.,* 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, *e.g.,* an anti-human antibody) conjugated to an enzymatic substrate (*e.g.,* horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that may be modified to increase the signal detected and to reduce the background noise. For further discussion regarding western blot protocols see, *e.g.,* Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that may be modified to increase the signal detected as well as other variations of ELISAs known in the art. For further discussion regarding ELISAs see, *e.g.,* Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction may be determined by competitive binding assays. One example of a competitive binding assay is a radioimmunoassay comprising the incubation of labeled antigen (*e.g.,* 3H or 125I) with the antibody of interest in the presence of increasing amounts of unlabeled antigen, and the detection of the antibody bound to the labeled antigen. The affinity of the antibody of interest for a particular antigen and the binding off-rates may be determined from the data by scatchard plot analysis. Competition with a second antibody may also be determined using radioimmunoassays. In this case, the antigen is incubated with antibody of interest conjugated to a labeled compound (e.g., 3H or 125I) in the presence of increasing amounts of an unlabeled second antibody.

The ability of an antibody to preferentially and specifically bind one antigen compared to another antigen may be determined using any method known in the art. By way of non-limiting example, an antibody may be considered to bind a first antigen preferentially if it binds said first antigen with a dissociation constant (K_{D}) that is less than the antibody's K_{D} for the second antigen. In another non-limiting embodiment, an antibody may be considered to bind a first antigen preferentially if it binds said first antigen with an affinity (*i.e*., K_{D}) that is at least one order of magnitude less than the antibody's K_{D} for the second antigen. In another non-limiting embodiment, an antibody may be considered to bind a first antigen preferentially if it binds said first antigen with an affinity (*i.e.,* K_{D}) that is at least two orders of magnitude less than the antibody's K_{D} for the second antigen.

In another non-limiting embodiment, an antibody may be considered to bind a first antigen preferentially if it binds said first antigen with an off rate (K_{off}) that is less than the antibody's K_{off} for the second antigen. In another non-limiting embodiment, an antibody may be considered to bind a first antigen preferentially if it binds said first antigen with a K_{off} that is at least one order of magnitude less than the antibody's K_{off} for the second antigen. In another non-limiting embodiment, an antibody may be considered to bind a first antigen preferentially if it binds said first antigen with a K_{off} that is at least two orders of magnitude less than the antibody's K_{off} for the second antigen.

Antibodies of the present invention may also be described or specified in terms of their cross-reactivity. Antibodies that do not bind any other analog, ortholog, or homolog of a polypeptide of the present invention are included. Antibodies that bind polypeptides with at least 100%, at least 99%, at least 98%, at least 97%, at least 96%, at least 95%, at least 94%, at least 93%, at least 92%, at least 91%, at least 90%, at least 80%, at least 70%, at least 60%, and at least 50% identity (as calculated using methods known in the art and described herein) to a polypeptide of the present invention are also included in the present invention.

Antibodies that do not bind polypeptides with less than 100%, less than 99%, less than 98%, less than 97%, less than 96%, less than 95%, less than 94%, less than 93%, less than 92%, less than 91 %, less than 90%, less than 80%, less than 70%, less than 60%, and less than 50% identity (as calculated using methods known in the art and described herein) to a polypeptide of the present invention are also included in the present invention. Further included in the present invention are antibodies which bind polypeptides encoded by polynucleotides which hybridize to a polynucleotide of the present invention under stringent hybridization conditions (as described herein). Antibodies of the present invention may also be described or specified in terms of their binding affinity to a polypeptide of the invention.

### Therapeutic uses

Disclosed are methods for the diagnosis and treatment of bone disorders. The methods may comprise the use of a binding composition specific for a polypeptide of MDL-1, e.g., an antibody or antigen binding fragment thereof or a soluble MDL-1 protein. Control binding compositions are also provided, e.g., control antibodies, see, *e.g.,* Lacey et al. (2003) Arthritis Rheum. 48:103-109; Choy and Panayi (2001) New Engl. J. Med. 344:907-916; Greaves and Weinstein (1995) New Engl. J. Med. 332:581-588; Robert and Kupper (1999) New Engl. J. Med. 341:1817-1828; Lebwohl (2003) Lancet 361:1197-1204.

In certain embodiments an MDL-1 antagonists, including antibodies specific for MDL-1 protein are used to inhibit bone resorption, including osteoclast formation and activation. The MDL-1 antagonists may also be administered to a subject to induce bone formation, including osteoblast activation. To induce bone formation, MDL-1 antagonists may be administered alone or in conjunction with additional standard of care therapies, as described below.

Methods for co-administration or treatment with a second therapeutic agent, *e.g.,* a cytokine, chemotherapeutic agent, antibiotic, or radiation, are well known in the art (Hardman, et al. (eds.) (2001) Goodman and Gilman's The Pharmacological Basis of Therapeutics, 10th ed., McGraw-Hill, New York, NY; Poole and Peterson (eds.) (2001) Pharmacotherapeutics for Advanced Practice:A Practical Approach, Lippincott, Williams & Wilkins, Phila., PA; Chabner and Longo (eds.) (2001) Cancer Chemotherapy and Biotherapy, Lippincott, Williams & Wilkins, Phila., PA).

Examples of such additional therapeutics include an agent that treats osteoclast-associated disorders, a chemotherapeutic agent, an interferon class drug such as interferon-alpha (e.g., from Amarillo Biosciences, Inc.), IFN-β-1a (REBIF® and AVONEX®) or IFN-β-1b (B ETASERON®), an oligopeptide such as glatiramer acetate (COPAXONE®), an agent blocking CD40-CD40 ligand, a cytotoxic or immunosuppressive agent (such as mitoxantrone (N OVANTRONE®), methotrexate, cyclophosphamide, chlorambucil, leflunomide, and azathioprine), intravenous immunoglobulin (gamma globulin), lymphocyte-depleting therapy (e.g., mitoxantrone, cyclophosphamide, CAMPATH® antibodies, anti-CD4, cladribine, total body irradiation, bone marrow transplantation, integrin antagonist or antibody (e.g., an LFA-1 antibody such as efalizumab/RAPTIVA® commercially available from Genentech, or an alpha 4 integrin antibody such as natalizumab/TYSABRI® available from Biogen Idec, or others as noted above), steroid such as corticosteroid (e.g., methylprednisolone such as S OLU-MEDROL® methylprednisolone sodium succinate for injection, prednisone such as low-dose prednisone, dexamethasone, or glucocorticoid, e.g., via joint injection, including systemic corticosteroid therapy), non-lymphocyte-depleting immunosuppressive therapy (e.g., MMF or cyclosporine), cholesterol-lowering drug of the "statin" class (which includes cerivastatin (BAYCOL®), fluvastatin (L ESCOL®), atorvastatin (LIPITOR®), lovastatin (MEVACOR®), pravastatin (P RAVACHOL®), and simvastatin (ZOCOR®)), estradiol, testosterone (optionally at elevated dosages; Stuve et al. Necrology 8:290-301 (2002)), androgen, hormone-replacement therapy, a TNF inhibitor such as an antibody to TNF-α, a disease-modifying anti-rheumatic drug (DMARD), a non-steroidal anti-inflammatory drug (NSAID), plasmapheresis or plasma exchange, trimethoprim-sulfamethoxazole (BACTRIM®, SEPTRA®), mycophenolate mofetil, H2-blockers or proton-pump inhibitors (during the use of potentially ulcerogenic immunosuppressive therapy), levothyroxine, cyclosporin A (e.g. SANDIMMUNE®), somatastatin analogue, cytokine, anti-metabolite, immunosuppressive agent, rehabilitative surgery, radioiodine, thyroidectomy, BAFF antagonist such as BAFF or BR3 antibodies or immunoadhesins, anti-CD40 receptor or anti-CD40 ligand (CD154), anti-IL-6 receptor antagonist/antibody, a B-cell surface antagonist or antibody such as a humanized or human CD20 antibody, IL-17 and/or IL-23 antibodies, etc. Also included are combination therapies using bisphosphonates (e.g., alendronate, ibandronate, risedronate, risedronate with calcium carbonate, zoledronic acid) and Cathepsin K inhibitors.

An effective amount of therapeutic will decrease the symptoms typically by at least 10%; usually by at least 20%; preferably at least 30%; more preferably at least 40%, and most preferably by at least 50%.

Formulations of therapeutic agents may be prepared for storage by mixing with physiologically acceptable carriers, excipients, or stabilizers in the form of, e.g., lyophilized powders, slurries, aqueous solutions or suspensions, see, *e.g.,* Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY,

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, *e.g.,* the inflammation or level of inflammatory cytokines produced. Preferably, a biologic that will be used is derived from the same species as the animal targeted for treatment, thereby minimizing a humoral response to the reagent.

An effective amount for a particular patient may vary depending on factors such as the condition being treated, the overall health of the patient, the method route and dose of administration and the severity of side affects. When in combination, an effective amount is in ratio to a combination of components and the effect is not limited to individual components alone. Guidance for methods of treatment and diagnosis is available (Maynard, et al. (1996) A Handbook of SOPs for Good Clinical Practice, Interpharm Press, Boca Raton, FL; Dent (2001) Good Laboratory and Good Clinical Practice, Urch Publ., London, UK).

Also disclosed is a kit comprising a cell and a compartment, a kit comprising a cell and a reagent, a kit comprising a cell and instructions for use or disposal, as well as a kit comprising a cell, compartment, and a reagent.

### Pharmaceutical Compositions

The antibody molecules of the invention may be administered, preferably for therapeutic purposes, to a subject, preferably in a Pharmaceutical composition. Preferably, a pharmaceutical composition includes a pharmaceutically acceptable carrier. The antibody molecules may be used therapeutically (e.g., in a pharmaceutical composition) to target the MDL-1 receptor and, thereby, to treat any medical condition caused or mediated by the receptor.

Pharmaceutically acceptable carriers are conventional and very well known in the art. Examples include aqueous and nonaqueous carriers, stabilizers, antioxidants, solvents, dispersion media, coatings, antimicrobial agents, buffers, serum proteins, isotonic and absorption delaying agents, and the like that are physiologically compatible. Preferably, the carrier is suitable for injection into a subject's body. Generally, compositions useful for parenteral administration of such drugs arc well known; *e.g.,* Remington's Pharmaceutical Science, 17th Ed. (Mack Publishing Company, Easton, PA, 1990).

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

The pharmaceutical compositions of the invention may be administered in conjunction with a second pharmaceutical composition or substance. When a combination therapy is used, both compositions may be formulated into a single composition for simultaneous delivery or formulated separately into two or more compositions (*e.g.,* a kit).

Analgesics may include aspirin, acetominophen, codein, morphine, aponorphine, normorphine, etorphine, buprenorphine, hydrocodone, racemorphan, levorphanol, butorphand, methadone, demerol, ibuprofen or oxycodone.

Pharmaceutical compositions of the invention may also include other types of substances, including small organic molecules and inhibitory ligand analogs, which may be identified using the assays described herein.

The formulations may conveniently be presented in unit dosage form and may be prepared by any methods well known in the art of pharmacy. See, *e.g.,* Gilman et al. (eds.) (1990),_The Pharmacological Bases of Therapeutics, 8th Ed., Pergamon Press; and Remington's Pharmaceutical Sciences, supra, Easton, Penn.; Avis et al. (eds.) (1993) Pharmaceutical Dosage Forms: Parenteral Medications Dekker, New York; Lieberman et al. (eds.) (1990) Pharmaceutical Dosage Forms: Tablets Dekker, New York; and Lieberman et al. (eds.) (1990), Pharmaceutical Dosage Forms: Disperse Systems Dekker, New York.

A further formulation and delivery method herein involves that described, for example, in WO 2004/078140, including the ENHANZE™ drug delivery technology (Halozyme Inc.). This technology is based on a recombinant human hyaluronidase (rHuPH20). rHuPH20 is a recombinant form of the naturally occurring human enzyme approved by the FDA that temporarily clears space in the matrix of tissues such as skin. That is, the enzyme has the ability to break down hyaluronic acid (HA), the space-filling "gel"-like substance that is a major component of tissues throughout the body. This clearing activity is expected to allow rHuPH20 to improve drug delivery and bioavailability of the therapeutic by enhancing the entry of therapeutic molecules through the subcutaneous space. Hence, when combined or co-formulated with certain injectable drugs, this technology can act as a "molecular machete" to facilitate the penetration and dispersion of these drugs by temporarily opening flow channels under the skin. Molecules as large as 200 nanometers may pass freely through the perforated extracellular matrix, which recovers its normal density within approximately 24 hours, leading to a drug delivery platform that docs not permanently alter the architecture of the skin.

Disclosed is a method of delivering the MDL-1 antibody herein to a tissue containing excess amounts of glycosaminoglycan, comprising administering a hyaluronidase glycoprotein (sHASEGP) (this protein comprising a neutral active soluble hyaluronidase polypeptide and at least one N-linked sugar moiety, wherein the N-linked sugar moiety is covalently attached to an asparagine residue of the polypeptide) to the tissue in an amount sufficient to degrade glycosaminoglycans sufficiently to open channels less than about 500 nanometers in diameter; and administering the antibody or soluble protein to the tissue comprising the degraded glycosaminoglycans.

Also disclosed is a method for increasing the diffusion of an antibody or soluble protein herein that is administered to a subject comprising administering to the subject a sHASEGP polypeptide in an amount sufficient to open or to form channels smaller than the diameter of the antibody and administering the antibody, whereby the diffusion of the therapeutic substance is increased. The sHASEGP and antibody may be administered separately or simultaneously in one formulation, and consecutively in either order or at the same time.

The dosage regimen involved in a therapeutic application may be determined by a physician, considering various factors which may modify the action of the therapeutic substance, *e.g.,* the condition, body weight, sex and diet of the patient, the severity of any infection, time of administration, and other clinical factors.

Often, treatment dosages are titrated upward from a low level to optimize safety and efficacy. Dosages may be adjusted to account for the smaller molecular sizes and possibly decreased half-lives (clearance times) following administration.

Typical protocols for the therapeutic administration of such substances are well known in the art. Pharmaceutical compositions of the invention may be administered, for example, by parenteral routes (*e.g.,* intravenous injection, intramuscular injection, subcutaneous injection, intratumoral injection or by infusion) or by a non-parenteral route (*e.g*., oral administration, pulmonary administration or topical administration).

Compositions may be administered with medical devices known in the art. For example, in a preferred embodiment, a pharmaceutical composition of the invention may be administered by injection with a hypodermic needle.

The pharmaceutical compositions of the invention may also be administered with a needleless hypodermic injection device; such as the devices disclosed in U.S. Patent Nos. 5,399,163; 5,383,851; 5,312,335; 5,064,413; 4,941,880; 4,790,824 or 4,596,556.

Examples of well-known implants and modules useful in the present invention include: U.S. Patent No. 4,487,603, which discloses an implantable micro-infusion pump for dispensing medication at a controlled rate; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments.

### EXAMPLES

The following Examples exemplify the present invention and should not be construed to limit the broad scope of the invention.

### I. General Methods

Some of the standard methods are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. *See, e.g.,* Ausubel, et al. (1987 and periodic supplements); Deutscher (1990) "Guide to Protein Purification" in Meth. Enzymol., vol. 182, and other volumes in this series; and manufacturer's literature on use of protein purification products, *e.g.,* Pharmacia, Piscataway, N.J., or Bio-Rad, Richmond, CA. Combination with recombinant techniques allow fusion to appropriate segments, *e.g.,* to a FLAG sequence or an equivalent which can be fused via a protease-removable sequence. *See, e.g.,* Hochuli (1990) "Purification of Recombinant Proteins with Metal Chelate Absorbent" in Setlow (ed.) Genetic Engineering, Principle and Methods 12:87-98, Plenum Press, N.Y.; and Crowe, et al. (1992) QIAexpress: The High Level Expression & Protein Purification System, Qiagen, Inc., Chatsworth, CA.

Computer sequence analysis is performed, *e.g.,* using available software programs, including those from the GCG (U. Wisconsin) and GenBank sources. Public sequence databases were also used, *e.g.,* from GenBank and others.

### II. Antagonists and Antibodies

Anti-mouse MDL-1 agonist antibodies (e.g., DX163, mouse IgG1) were generated from a BALB/c mouse immunized with a fusion protein consisting of the extracellular domain of human MDL-1 gene fused to the Fc domain of hIg, as decribed previously (see, e.g., Wright et al. (2003) J Immunol. 171:3034-3046). The extracellular domain of the fusion protein contained the C-type lectin domain and corresponded to the amino acid positions 26-187 of human MDL-1 (GenBank accession number # BC112099; SEQ ID NO: 2).

Because the ligand for MDL-1 receptor has not been identified, a soluble form of MDL-1 that could bind the endogenous ligand and inhibit the in vivo activities of MDL-1 was generated. This soluble MDL-1 antagonist is composed of the extracellular (163 amino acids) portion of the long form of mouse MDL-1 (GenBank accession number AA186015; SEQ ID NO: 4) was ligated into a pCMV1 expression plasmid containing the Fc portion of mIgG2a that has been mutated (L to E; see, e.g., Duncan et al. (1988) Nature 332:563-564) for low FcγRI binding properties. Protein was expressed in 293 freestyle cells.

### III. Cell Stimulation

For MDL-1 activation, freshly isolated neutrophils resuspended (10⁷ cells/ml) in RPMI 1640 with 10% of fetal calf serum were incubated with anti-MDL-1 antibody (10 µg/ml) or control mouse IgG1 for 1h at 37°C and 5% CO₂ in 96-well plates (Nunc, Denmark). After two washes with RPMI, cell-bound anti-hMDL-1 antibodies were cross-linked with 9 µg/ml of F(ab')₂ fragment goat anti-mouse IgG (H+L) antibody for 30 min at 37°C and 5% CO₂. Cells were then washed twice and incubated with 20 µg/ml of chrompure mouse IgG antibody for 20 min at 4°C to block unbound cross-linking antibody. In some experiments, different doses of anti-MDL-1 antibody and m IgG1 isotype control were tested (0.1 ng/ml - 20 µg/ml). Following MDL-1 or mIgG1 incubation, cells will then treated for 22 hours with 0.2 ng/mL LPS or medium.

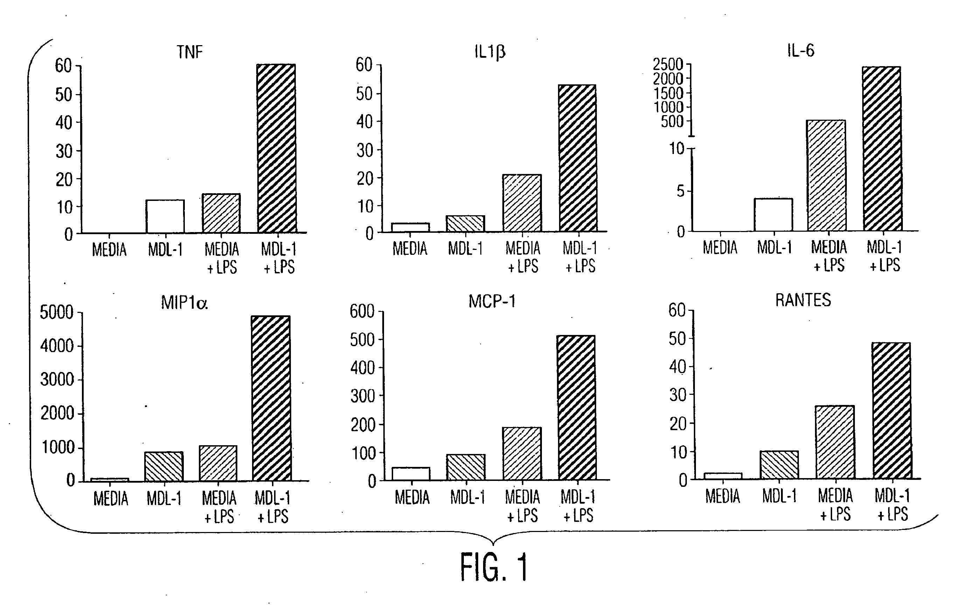

Figure 1 shows that activation of MDL-1 augments the LPS induced release of inflammatory mediators.

### IV. Collagen Induced Arthritis

In these experiments, B10RIII mice were immunized at the base of the tail with 100ug of Bovine collagen in Complete Freund's adjuvant. At day 26 and day 32 mice were given intraperitoneal injections of 50 ug or 500 ug of isotype control antibody or DX163 (rat anti-mouse MDL-1 agonist antibody). Mice were then monitored and scored for the development of arthritis in each paw based on a four point disease score scale. Clinical scores are based on paw swelling per foot. Score 1= 1 toe, 2= two or more toes 3= entire paw swelling. Maximum clinical score per mouse is 12.

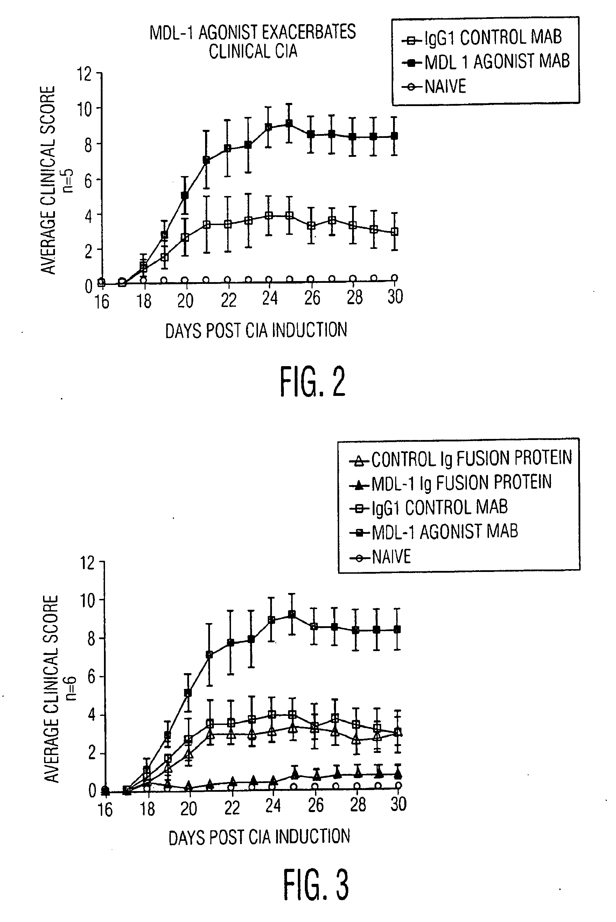

MDL-1-Ig fusion treated mice were highly resistant to both CIA compared to controls and this contrasts with the enhanced disease observed in anti-MDL-1 agonist treated mice Figure 2 shows exacerbation of CIA with administration of the MDL-1 agonist antibody, DX163. Figure 3 shows inhibition of CAIA by the MDL-1 antagonist MDL-1-Ig fusion protein.

### V. Collagen Antibody Induced Arthritis

B10 RIII mice (n=5 per group) or MDL-1 -/- KO mice were given 800 ug of Chemicon's arthrogen CIA cocktail iv to induce collagen antibody induced arthritis on Day 0. Mice were subsequently given a single 0.5 mg dose sc of either IgG1 isotype control antibody or DX163 (rat anti-mouse MDL-1.). Clinical score is based on paw swelling per foot. Score 1= 1 toe, 2= two or more toes 3= entire paw swelling. Maximum clinical score per mouse is 12.

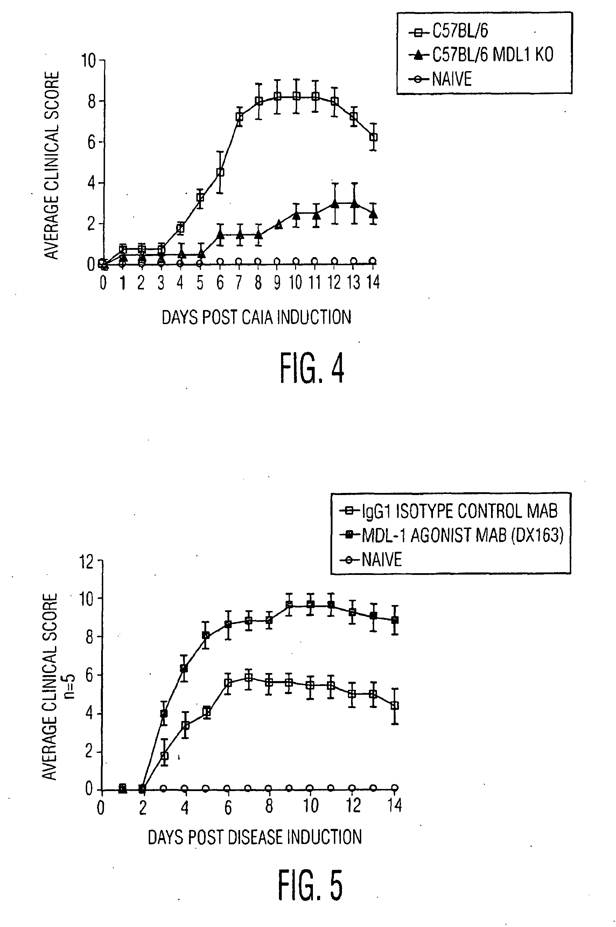

Mice treated with DX163 have increased scores for all parameters examined. H&E sections comparing naive, IgG1 isotype treated and DX163 treated animals showed marked increases in neturophil infiltrate/invasion, bone erosion, and pannus formation in DX163 treated animals. Figures 4 shows lower CAIA scores in MDL-1 KO mice. Figure 5 confirms that agonizing MDL-1 activity with DX163 can exacerbate development of autoimmune arthritis.

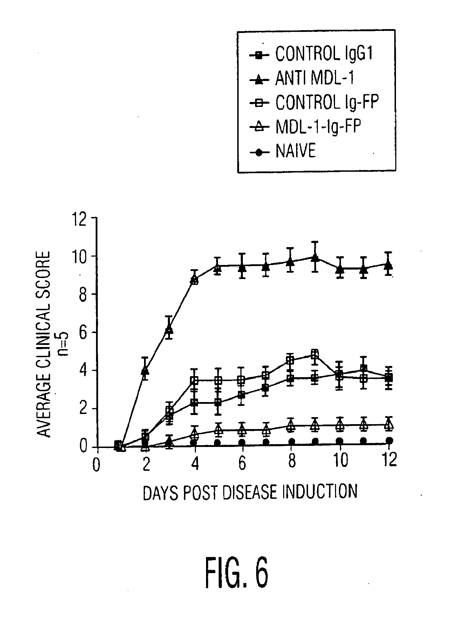

MDL-1 Ig fusion protein was also administered in the same model. B10 RIII mice (n=5 per group) were given 1000 ug of Chemicon's arthrogen CIA cocktail iv to induce collagen antibody induced arthritis on Day 0. Mice were given 0.5 mg dose sc of either IgG1 isotype control antibody, DX163 (rat anti-mouse MDL-1), Ig control, or MDL-1 Ig fusion protein on day 0 and day 2. Foot pad swelling was measure as described above. Figure 6 shows inhibition of CAIA with MDL-1-Ig fusion protein.

### VI. Histopathological Assessment

The paws were removed, fixed, decalcified in Cal-EX II (Fisher Scientific) for 7 days, and embedded in paraffin. Subsequently, the paw sections were made and stained with hematoxylin and eosin, and examined by light microscopy. A histological analysis of synovial, bone and cartilage tissues was performed as a blind test. Scoring of leucocyte infiltration, and more specifically, the percentage of infiltrated PMNs was examined in the synovium and joint space. In addition to cell recruitment, Pannus formation was also evaluated. On bone and cartilage tissues, destruction of cartilage and cortical bone erosions were scored. The severity was graded on a scale of 0-4. Comparisons between groups were assessed with the two-tailed unpaired t test with a p value of <0.05 considered as statistically significant. Calculations were performed with the statistical package, GraphPad Prism 4 (GraphPad Software, San Diego, CA).

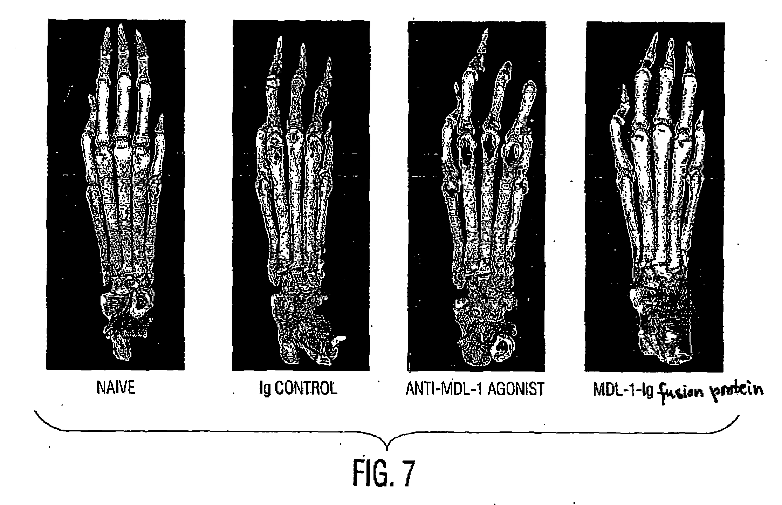

Figure 7 shows paws from B10RIII mice that were scanned with GE explore CT scanner. Joints were compared between naive and CAIA induced mice treated with either MDL-1 Ig fusion protein or DX163. Bone samples were taken on day 12 of experiment. Anti-MDL-1 agonist treatment induced considerable cortical bone destruction compared to the moderate damage in the Ig control group. In contrast, MDL-1-Ig fusion treated mice showed bone integrity and density comparable to naive mice confirming that blocking MDL-1 signaling prevented bone resorption.

### VII. RT-PCR and Affymetrix MicroArray Analyses

RNA was extracted from the paws and complementary DNAs were prepared as described (Murphy, 2002) and used for RT-PCR and Affymetrix gene expression analysis. For RT-PCR, gene expression of a range of genes was analyzed using the GeneAmp 5700 Sequence Detection System (Applied Biosystems). The housekeeping gene, ubiquitin, was used to normalize the analysis. For Affymetrix analysis, DNase-treated total RNA was synthesized into biotinylated cRNA probe using one-cycle target labeling and IVT labeling (Affymetrix, Inc., Santa Clara, CA) according to manufacturer's instructions. 15 µg of biotinylated cRNA probe from each sample was hybridized onto murine MOE430 2.0 Affymetrix Microarray chips. The hybridized chips were then washed using the Affymetrix GeneChip Fluidics 400 station and scanned in the Affymetrix GeneChip Scanner 3000 (Affymetrix, Inc., Santa Clara, CA) according to manufacturer's instructions. The quality of cRNA probe synthesis and efficiency of hybridization was analyzed in the GeneChip Operating System for each Affymetrix chip once scanning was complete. The chip data was then normalized using MAS 5.

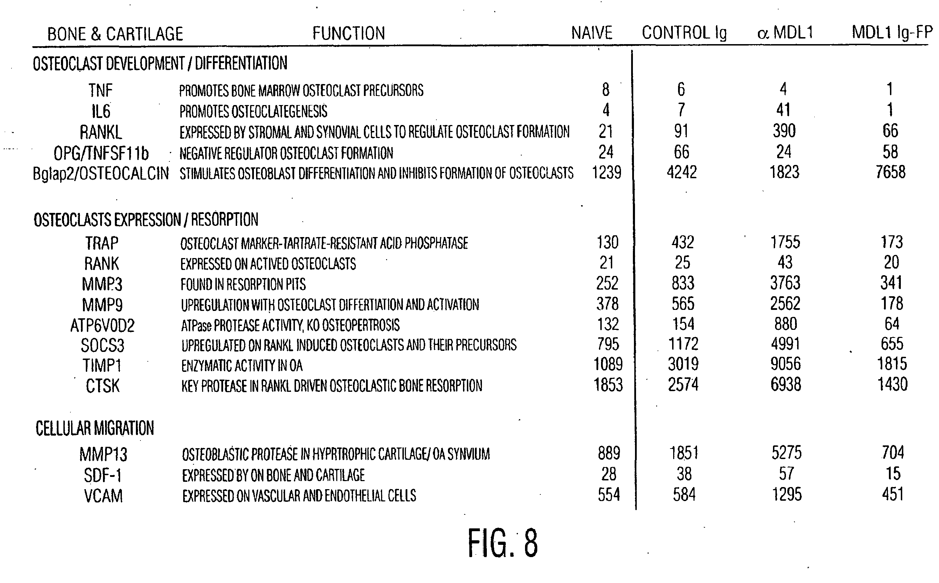

To investigate the immune pathways regulated by MDL-1 activation, the expression of proinflammatory cytokines, chemokines, cell adhesion molecules, myeloid cell markers was assessed by quantitative RT-PCR assay. The osteoclast genes (ATP6VOD2 and Cathepsin K) were assessed by Affymetrix analysis. On day 4 after induction of CAIA, pro-inflammatory genes such as IL-1β, IL-6 and TNFα were abundantly expressed in all the arthritic paws from anti-MDL-1 agonist treated mice compared to Ig controls. Importantly, genes associated with bone destruction such as RANKL, matrix metaloprotease 9 (MMP9) and TRAP were up-regulated in paws after anti-MDL-1 agonist treatment and down-regulated in MDL-1-Ig fusion treatment (see Figure 8). The gene expression results together with the histopathology findings suggest that MDL-1 plays a role in recruiting and activating inflammatory macrophages and neutrophils, which mediates synovial tissue injury. In addition, the induction of osteoclast-specific genes and the X ray microCT findings suggest the MDL-1-DAP12 pathway might also have a role in bone metabolism.

The expression results were transferred into Spotfire DecisionSite (Spotfire, Somerville, MA) for data filtering and graph analysis. Probe sets were filtered out if they had a signal strength less than 20 and a detection call of "A" (Absent) across all samples. Comparison lists for samples were generated using fold change as a signed ratio. These lists were used for further pathway analysis in Ingenuity Systems (Ingenuity Systems, Inc., Redwood City, CA).

### VIII. In vitro Osteoclast Cultures

Bone marrow cells derived from 12 week old C57BL/6 or B10RIII mice were cultured for 2 days in alpha-mem media (Gibco) with 50 ng/mL of recombinant MSCF (R&D). Loosely adherent cells were then plated at 4-8 x 10E5/ mL in tissue culture plates with or without the addition on bovine bone chips (Nordic Biosciences, NY). Cells were treated with a dose range of RANK-L (0-100ng/mL) and MDL-1 agonist or control antibodies at (0-50 ug/mL). Conditions were evaluated for osteoclast formation kinetics, cell culture cytokine profile by Luminex (Linco Inc.), RT-PCR analysis of cell cultures, TRAP staining (Alkaline phosphatase staining kit, Sigma), and nuclear extract ELISA assay (TransAM NFATcl transcription factor assay kit, Active Motif).

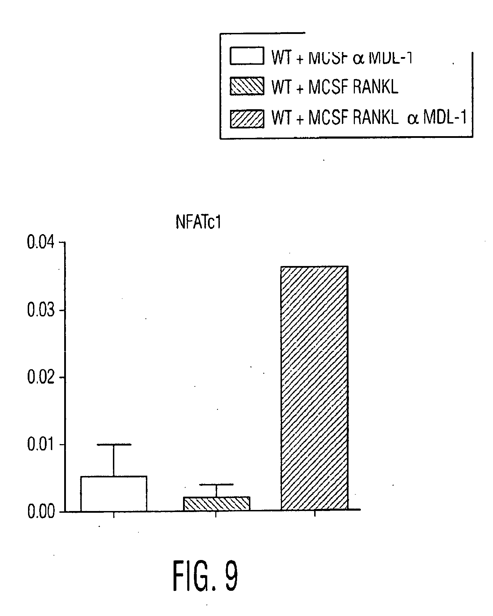

TRAP staining of MCSF-cultured bone marrow cells indicated that RANK-L and MDL-1 agonist antibody treatment increased osteoclast formation and enhanced bone resorption. Figure 9 showed that treatment with RANK-L in combination with anti-MDL-1 agonist antibody increased expression of the osteoclast "master transcription regulator" NFATc1 which controls the downstream transcription of DC STAMP, ATP6VOD2 (required for cell fusions), Cathepsin K, MMP9, ATP6I, CIC7 (required for bone resorption).

### IX. Statistical Analysis

Comparisons between groups were assessed with the two-tailed unpaired t test. A p value of <0.05 was considered as statistically significant. Calculations were performed with a statistical package, GraphPad Prism 4 (GraphPad Software, San Diego, CA).

## Claims

1. An MDL-1 antagonist which is an antibody or antibody fragment thereof that specifically binds MDL-1 SEQ ID NO:2 or 4 or a soluble MDL-1 protein SEQ ID NO:2 or 4, for use in the diagnosis or treatment of a disease **characterized by** bone loss.

2. An MDL-1 antagonist for use according to claim 1, wherein the antibody is humanized.

3. An MDL-1 antagonist for use according to claim 1, wherein the antibody is fully human.

4. An MDL-1 antagonist for use according to claim 1, wherein the antibody is chimeric.

5. An MDL-1 antagonist for use according to claim 1, wherein the antibody fragment is a Fab, Fab2, or Fv antibody fragment.

6. An MDL-1 antagonist for use according to claim 1, wherein the antibody, antibody fragment or soluble MDL-1 protein is conjugated to another chemical moiety.

7. An MDL-1 antagonist for use according to claim 6, wherein the chemical moiety is polyethylene glycol (PEG).

8. An MDL-1 antagonist according for use to claim 1, wherein the soluble MDL-1 protein is fused to an Fc portion of an antibody molecule.

9. Use of an MDL-1 antagonist as defined in any preceding claim for the manufacture of a medicament for diagnosing or treating a disease **characterized by** bone loss.

10. An MDL-1 antagonist for use according to of any one of claims 1 to 8 which is for co-administration with a second therapeutic agent.

## Patentansprüche

1. Ein MDL-1-Antagonist, der ein Antikörper oder Antikörperfragment davon ist, der/das spezifisch MDL-1 der SEQ ID NO: 2 oder 4 oder ein lösliches MDL-1-Protein der SEQ ID NO:2 oder 4 bindet, zur Verwendung bei der Diagnose oder Behandlung einer Erkrankung, die durch Knochenschwund gekennzeichnet ist.

2. Ein MDL-1-Antagonist zur Verwendung gemäß Anspruch 1, wobei der Antikörper humanisiert ist.

3. Ein MDL-1-Antagonist zur Verwendung gemäß Anspruch 1, wobei der Antikörper vollständig human ist.

4. Ein MDL-1-Antagonist zur Verwendung gemäß Anspruch 1, wobei der Antikörper chimär ist.

5. Ein MDL-1-Antagonist zur Verwendung gemäß Anspruch 1, wobei das Antikörperfragment ein Fab-, Fab2- oder Fv-Antikörperfragment ist.

6. Ein MDL-1-Antagonist zur Verwendung gemäß Anspruch 1, wobei der Antikörper, das Antikörperfragment oder das lösliche MDL-1-Protein mit einem weiteren chemischen Rest konjugiert ist.

7. Ein MDL-1-Antagonist zur Verwendung gemäß Anspruch 6, wobei der chemische Rest Polyethyleneglycol (PEG) ist.

8. Ein MDL-1-Antagonist zur Verwendung gemäß Anspruch 1, wobei das lösliche MDL-1-Protein an einen Fc-Teil eines Antikörpermoleküls fusioniert ist.

9. Verwendung eines MDL-1-Antagonisten wie in einem vorhergehenden Anspruch definiert zur Herstellung eines Medikaments zur Diagnose oder Behandlung einer Erkrankung, die durch Knochenschwund gekennzeichnet ist.

10. Ein MDL-1-Antagonist zur Verwendung gemäß einem der Ansprüche 1 bis 8 zur gemeinsamen Verabreichung mit einem zweiten therapeutischen Mittel.

## Revendications

1. Antagoniste de MDL-1 qui est un anticorps ou un fragment d'anticorps de celui-ci qui se lie spécifiquement à la SEQ ID NO:2 ou 4 de MDL-1 ou à la SEQ ID NO:2 ou 4 d'une protéine soluble de MDL-1 pour une utilisation dans le diagnostic ou le traitement d'une maladie **caractérisée par** l'ostéoporose.

2. Antagoniste de MDL-1 pour une utilisation selon la revendication 1, dans lequel l'anticorps est humanisé.

3. Antagoniste de MDL-1 pour une utilisation selon la revendication 1, dans lequel l'anticorps est entièrement humain.

4. Antagoniste de MDL-1 pour une utilisation selon la revendication 1, dans lequel l'anticorps est chimérique.

5. Antagoniste de MDL-1 pour une utilisation selon la revendication 1, dans lequel le fragment d'anticorps est un Fab, Fab2 ou un fragment d'anticorps Fv.

6. Antagoniste de MDL-1 pour une utilisation selon la revendication 1, dans lequel l'anticorps, le fragment d'anticorps ou la protéine soluble de MDL-1 est conjuguée à un autre groupe fonctionnel.

7. Antagoniste de MDL-1 pour une utilisation selon la revendication 6, dans lequel le groupe fonctionnel est le polyéthylène glycol (PEG).

8. Antagoniste de MDL-1 pour une utilisation selon la revendication 1, dans lequel la protéine de MDL-1 soluble est fusionnée à une portion Fc d'une molécule d'anticorps.

9. Utilisation d'un antagoniste de MDL-1 tel que défini dans l'une quelconque des revendications précédentes pour la fabrication d'un médicament pour diagnostiquer ou pour traiter une maladie **caractérisée par** l'ostéoporose.

10. Antagoniste de MDL-1 pour une utilisation selon l'une quelconque des revendications 1 à 8 destiné à être coadministré avec un deuxième agent thérapeutique.


### SEQUENCE LISTING

<110> Schering Corporation Bigler, Michael E Cua, Daniel J Joyce-Shaikh, Barbara Phillips, Joseph H
<120> MDL-1 USES
<130> BP06654
<150> 60/947,314 <151> 2007-06-29
<160> 4
<170> PatentIn version 3.5
<210> 1
   <211> 996
   <212> DNA
   <213> Homo sapiens
<400> 1
   ggcttagcgt ggtcgcggcc gaggtggcaa aaggagcata ttctcaggag acggggcccc      60
   tgcctgccac accaagcatt aggccaccag gaagaccccc atctgcaagc aagcctagcc     120
   ttccagggag aaagaggcct ctgcagctcc ttcatcatga actggcacat gatcatctct     180
   gggcttattg tggtagtgct taaagttgtt ggaatgacct tatttctact ttatttccca     240
   cagattttta acaaaagtaa cgatggtttc accaccacca ggagctatgg aacagtctca     300
   cagatttttg ggagcagttc cccaagtccc aacggcttca ttaccacaag gagctatgga     360
   acagtctgcc ccaaagactg ggaattttat caagcaagat gttttttctt atccacttct      420
   gaatcatctt ggaatgaaag cagggacttt tgcaaaggaa aaggatccac attggcaatt     480
   gtcaacacgc cagagaaact gtttcttcag gacataactg atgctgagaa gtattttatt     540
   ggcttaattt accatcgtga agagaaaagg tggcgttgga tcaacaactc tgtgttcaat     600
   ggcaatgtta ccaatcagaa tcagaatttc aactgtgcga ccattggcct aacaaagacc     660
   tttgatgctg catcatgtga catcagctac cgcaggatct gtgagaagaa tgccaaatga     720
   tcacagttcc ctgtgacaag aactatactt gcaactcttt ttgaatccat aacaggtcgt     780
   actggccaat gattactttt acttacctat ctgtactacc agtagcggtc cttgcccatt     840
   tgggaaactg agcttctttc ttctgcactg ggggactgga tgctagccat ctccaggaga     900
   caggatcagt tttacggaaa caactcagtt agtatagaga tgaggtccgc ttctgtagta     960
   ccttccttca aataaagaaa tttggtacct gcccgg                                996
<210> 2
   <211> 188
   <212> PRT
   <213> Homo sapiens
<220>
   <221> DOMAIN
   <222> (26)..(188)
   <223> extracellular domain
<400> 2
   Met Asn Trp His Met Ile Ile Ser Gly Leu Ile Val Val Val Leu Lys
   1               5                   10                  15
   Val Val Gly Met Thr Leu Phe Leu Leu Tyr Phe Pro Gln Ile Phe Asn
               20                  25                  30
   Lys Ser Asn Asp Gly Phe Thr Thr Thr Arg Ser Tyr Gly Thr Val Ser
           35                  40                  45
   Gln Ile Phe Gly Ser Ser Ser Pro Ser Pro Asn Gly Phe Ile Thr Thr
       50                  55                  60
   Arg Ser Tyr Gly Thr Val Cys Pro Lys Asp Trp Glu Phe Tyr Gln Ala
   65                  70                  75                  80
   Arg Cys Phe Phe Leu Ser Thr Ser Glu Ser Ser Trp Asn Glu Ser Arg
                   85                  90                  95
   Asp Phe Cys Lys Gly Lys Gly Ser Thr Leu Ala Ile Val Asn Thr Pro
               100                 105                 110
   Glu Lys Leu Lys Phe Leu Gln Asp Ile Thr Asp Ala Glu Lys Tyr Phe
           115                 120                 125
   Ile Gly Leu Ile Tyr His Arg Glu Glu Lys Arg Trp Arg Trp Ile Asn
       130                 135                 140
   Asn Ser Val Phe Asn Gly Asn Val Thr Asn Gln Asn Gln Asn Phe Asn
   145                 150                 155                 160
   Cys Ala Thr Ile Gly Leu Thr Lys Thr Phe Asp Ala Ala Ser Cys Asp
                   165                 170                 175
   Ile Ser Tyr Arg Arg Ile Cys Glu Lys Asn Ala Lys
               180                 185
<210> 3
   <211> 896
   <212> DNA
   <213> Mus musculus
<400> 3
   aggacattac cgagcaggag catacatttc cagagcaagg agccctgctc gctgcaccga      60
   atatcttatc aaaaagactc ctatctgtat gccaacccag acttcccaga agagatcaga     120
   tccctgatcc cccatcatca tgaactggca catgatcatc tcggggctta tcgtagtagt     180
   gatcaaagtt gttggaatga cctttttctt gctgtatttc ccacaggttt ttggcaaaag     240
   taatgatggc ttcgtcccca cggagagcta cggaaccact agtgtgcaga atgtctcaca     300
   gatctttggg agaaatgacg aaagtaccat gcctacaagg agctatggaa cagtctgtcc     360
   cagaaactgg gattttcacc aaggaaaatg ctttttcttc tccttctccg aatcaccttg     420
   gaaagacagc atggattatt gtgcaacaca aggatccaca ctggcaattg tcaacactcc     480
   agagaaactg aagtatcttc aggacatagc tggtattgag aattacttta ttggtttggt     540
   acgtcagcct ggagagaaaa agtggcgctg gatcaacaac tctgtgttca atggcaatgt     600
   taccaatcag gaccagaact tcgactgtgt cactataggt ctgacgaaga catatgatgc     660
   tgcatcatgt gaagtcagct atcgctggat ctgcgaaatg aatgccaaat gatcatagat     720
   ctctacaaga gtgaattttt acagagctag caaaggagat tagttgtgac tgaaaccagc     780
   ccaggaaaat atagagcatc aaagactgtg cccatcttca taggtgggag ttccctattg     840
   aatcctcaaa gtcaattttg ttactccaca aacatcttac catagtaaaa ctccct         896
<210> 4
   <211> 190
   <212> PRT
   <213> Mus musculus
<220>
   <221> DOMAIN
   <222> (26) .. (190)
   <223> extracellular domain
<400> 4
   Met Asn Trp His Met Ile Ile Ser Gly Leu Ile Val Val Val Ile Lys
   1               5                   10                  15
   Val Val Gly Met Thr Phe Phe Leu Leu Tyr Phe Pro Gln Val Phe Gly
               20                  25                  30
   Lys Ser Asn Asp Gly Phe Val Pro Thr Glu Ser Tyr Gly Thr Thr Ser
           35                  40                  45
   Val Gln Asn Val Ser Gln Ile Phe Gly Arg Asn Asp Glu Ser Thr Met
       50                  55                  60
   Pro Thr Arg Ser Tyr Gly Thr Val Cys Pro Arg Asn Trp Asp Phe His
   65                  70                  75                  80
   Gln Gly Lys Cys Phe Phe Phe Ser Phe Ser Glu Ser Pro Trp Lys Asp
                   85                  90                  95
   Ser Met Asp Tyr Cys Ala Thr Gln Gly Ser Thr Leu Ala Ile Val Asn
               100                 105                 110
   Thr Pro Glu Lys Leu Lys Tyr Leu Gln Asp Ile Ala Gly Ile Glu Asn
           115                 120                 125
   Tyr Phe Ile Gly Leu Val Arg Gln Pro Gly Glu Lys Lys Trp Arg Trp
       130                 135                 140
   Ile Asn Asn Ser Val Phe Asn Gly Asn Val Thr Asn Gln Asp Gln Asn
   145                 150                 155                 160
   Phe Asp Cys Val Thr Ile Gly Leu Thr Lys Thr Tyr Asp Ala Ala Ser
                   165                 170                 175
   Cys Glu Val Ser Tyr Arg Trp Ile Cys Glu Met Asn Ala Lys
               180                 185                 190